# EUROPEAN PATENT SPECIFICATION

(11) **EP 2 968 437 B1**
(45) Date of publication and mention of the grant of the patent: **29.07.2020**
(21) Application number: 14714100.6
(22) Date of filing: 11.03.2014
(51) Int. Cl.: A61K 38/01, A61K 31/702, A61K 31/202, A61P 29/00, A61P 3/10, A61P 1/00

(54) **CASEIN HYDROLYSATE FOR USE IN TREATING INFLAMMATORY DISEASES**
KASEINHYDROLYSAT ZUR VERWENDUNG BEI DER BEHANDLUNG VON ENTZÜNDUNGSKRANKHEITEN
HYDROLYSAT DE CASÉINE POUR L'UTILISATION DANS LE TRAITEMENT DES MALADIES INFLAMMATOIRES

(30) Priority: 15.03.2013 US 201313838539
(43) Date of publication of application: 20.01.2016
(73) Proprietor: MJN U.S. Holdings LLC, Evansville, Indiana 47721 (US)
(72) Inventor: HONDMANN, Dirk, Winnetka, Illinois 60093 (US); VAN TOL, Eric A.F., NL-6824 MZ Arnhem (NL); GROSS, Gabriele, NL-6521 MR Nijmegen (NL); SCHOEMAKER, Marieke H., 3911 BH Rhenen (NL); LAMBERS, Teartse Tim, NL-6515 CA Nijmegen (NL)
(74) Representative: Cawdell, Karen Teresa
(86) International application number: PCT/US2014/023637
(87) International publication number: WO 2014/150571

(56) References cited:
- EP-A1- 2 332 428
- WO-A2-2005/081628
- US-A1- 2008 096 794
- US-A1- 2009 075 904
- MAO X Y ET AL: "Free-radical-scavenging and anti-inflammatory effect of yak milk casein before and after enzymatic hydrolysis", FOOD CHEMISTRY, ELSEVIER LTD, NL, vol. 126, no. 2, 15 May 2011 (2011-05-15), pages 484-490, XP027571813, ISSN: 0308-8146 [retrieved on 2010-12-22]

## Description

### TECHNICAL FIELD

The disclosure relates to a method for reducing a proinflammatory response using a casein hydrolysate.

### BACKGROUND ART

Proinflammatory response, or inflammation, is the biological response of the body to harmful stimuli such as pathogens, damaged cells or irritants.

The classical signs of acute inflammation are pain, heat, redness, swelling, and loss of function. Inflammation is a protective attempt by the organism to remove the injurious stimuli and to initiate the healing process. Inflammation is not a synonym for infection, even in cases where inflammation is caused by infection. Although infection is caused by a microorganism, inflammation is one of the responses of the organism to the pathogen. While acute inflammation is important to the immune response and prevents further destruction of tissue, chronic inappropriate inflammation can cause tissue destruction (neurodegenerative, cardiovascular, type 2 diabetes) and diseases such as hay fever, periodontitis, atherosclerosis, rheumatoid arthritis, type 1 diabetes, autoimmune disease, and even cancer (e.g., gallbladder carcinoma). Prolonged inflammation, or chronic inflammation, leads to a progressive shift in the type of cells present at the site of inflammation and is characterized by simultaneous destruction and healing of the tissue from the inflammatory process. The inflammatory response is part of the innate immune response, and employs cellular and plasma-derived agents (pathway) such as complement system, interferons (IFN), cytokines, lymphokines, monokines, prostaglandins and leukotrienes, platelet activating factor (PAF), histamine, and kinins (e.g. bradykinin associated with pain). Pro-inflammatory cytokines include IL-1(interleukin-1α and β), IL-6, IL-8, TNF-α (tumor necrosis factor alpha), and TNF-β (lymphotoxin α), as well as members of the IL20 family, IL33, LIF (leukocyte inhibitory factor), IFN-γ (interferon gamma), OSM (oncostatin M), CNTF (ciliary neurotrophic factor), TGF-β (transforming growth factor-beta), GM-CSF (granulocyte-macrophage colony stimulating factor), IL11, IL12, and IL18.

Autoimmune diseases arise from an inappropriate immune response of the body against substances and tissues normally present in the body. This may be restricted to certain organs (e.g. in autoimmune thyroiditis) or involve a particular tissue in different places (e.g. Goodpasture's disease which may affect the basement membrane in both the lung and the kidney). The treatment of autoimmune diseases is typically with immunosuppression medication that decreases the immune response.

Diabetes mellitus type 1 (type 1 diabetes, T1DM, formerly insulin dependent or juvenile diabetes) is a form of diabetes mellitus that results from autoimmune destruction of insulin-producing beta cells of the pancreas. The subsequent lack of insulin leads to increased blood and urine glucose. The classical symptoms are polyuria (frequent urination), polydipsia (increased thirst), polyphagia (increased hunger), and weight loss. Incidence varies from 8 to 17 per 100,000 in Northern Europe and the U.S. with a high of about 35 per 100,000 in Scandinavia to a low of 1 per 100,000 in Japan and China. Eventually, type 1 diabetes is fatal unless treated with insulin. Injection is the most common method of administering insulin although other methods are insulin pumps and inhaled insulin. Other alternatives are Pancreatic transplants that have been used and also pancreatic islet cell transplantation. Transplantation is experimental yet growing.

Coeliac disease is an autoimmune disorder of the small intestine that occurs in genetically predisposed people of all ages from middle infancy onward. Symptoms include pain and discomfort in the digestive tract, chronic constipation and diarrhea, failure to thrive (in children), and fatigue, but these may be absent, and symptoms in other organ systems have been described. Vitamin deficiencies are often noted in people with Coeliac disease due to the reduced ability of the small intestine to properly absorb nutrients from food. Increasingly, diagnoses are being made in asymptomatic persons as a result of increased screening; the condition is thought to affect between 1 in 1,750 and 1 in 105 people in the United States. Coeliac disease is caused by a reaction to gliadin, a prolamin (gluten protein) found in wheat, and similar proteins found in the crops of the tribe Triticeae (which includes other common grains such as barley and rye). Upon exposure to gliadin, and specifically to three peptides found in prolamins, the enzyme tissue transglutaminase modifies the protein, and the immune system cross-reacts with the small-bowel tissue, causing an inflammatory reaction. That leads to a truncating of the villi lining the small intestine (called villous atrophy). This interferes with the absorption of nutrients, because the intestinal villi are responsible for absorption. The only known effective treatment is a lifelong gluten-free diet. While the disease is caused by a reaction to wheat proteins, it is not the same as wheat allergy.

HLA-DQ2 (DQ2) is a serotype group within HLA-DQ (DQ) serotyping system. The serotype is determined by the antibody recognition of β2 subset of DQ β-chains. DQ2 represents the second highest risk factor for coeliac disease, the highest risk is a close family member with disease. Due to its link to coeliac disease, DQ2 has the highest association of any HLA serotype with autoimmune disease, close to 95% of all celiacs have DQ2, of that 30% have 2 copies of DQ2. Of the DQ2 homozygotes who eat wheat, lifelong risk is between 20 and 40% for coeliac disease. Juvenile diabetes (T1D) has a high association with DQ2.5 and there appears to be link between GSE and early onset male T1D. Anti-tTG antibodies are found elevated in a one-third of T1D patients, and there are indicators that Triticeae may be involved but the gluten protein is a type of globulin (Glb1). Recent studies indicate a combination of DQ2.5 and DQ8 (both acid peptide presenters) greatly increase the risk of adult onset Type 1 Diabetes and ambiguous type I/II Diabetes. HLA-DR3 plays a prominent role in autoimmune diabetes. However, DQ2 presence with DR3 decreases the age of onset and the severity of the autoimmune disorder.

HLA-DQ8 (DQ8) is a human leukocyte antigen serotype within the HLA-DQ (DQ) serotype group. DQ8 is a split antigen of the DQ3 broad antigen. DQ8 is determined by the antibody recognition of β8 and this generally detects the gene product of DQB1*0302. DQ8 is commonly linked to autoimmune disease in the human population. DQ8 is the second most predominant isoform linked to coeliac disease and the DQ most linked to juvenile diabetes. DQ8 increases the risk for rheumatoid arthritis and is linked to the primary risk locus for RA, HLA-DR4. DR4 also plays an important role in juvenile diabetes. While the DQ8.1 haplotype is associated with disease, there is no known association with the DQB1*0305, DQ8.4 or DQ8.5 haplotypes with autoimmune disease; however, this may be the result of lack of study in populations that carry these and the very low frequency. In Europe, DQ8 is associated with juvenile diabetes and coeliac disease. The highest risk factor for type 1 diabetes is the HLA DQ8/DQ2.5 phenotype. In parts of eastern Scandinavia both DQ2.5 and DQ8 are high increases frequencies of late onset Type I and ambiguous Type I/II diabetes. DQ8 is also found in many indigenous peoples of Asia, it was detected early on in the Bedoin population of Arabia where DQ2.5 is frequently absent, and in these instances DQ8 is solely associated HLA in Coeliac Disease.

Crohn's disease, also known as Crohn syndrome and regional enteritis, is a type of inflammatory bowel disease that may affect any part of the gastrointestinal tract from mouth to anus, causing a wide variety of symptoms. It primarily causes abdominal pain, diarrhea (which may be bloody if inflammation is at its worst), vomiting (can be continuous), or weight loss, but may also cause complications outside the gastrointestinal tract such as skin rashes, arthritis, inflammation of the eye, tiredness, and lack of concentration. Crohn's disease is caused by interactions between environmental, immunological and bacterial factors in genetically susceptible individuals. This results in a chronic inflammatory disorder, in which the body's immune system attacks the gastrointestinal tract possibly directed at microbial antigens. There is a genetic association with Crohn's disease, primarily with variations of the NOD2 gene and its protein, which senses bacterial cell walls. Siblings of affected individuals are at higher risk. Males and females are equally affected. Smokers are two times more likely to develop Crohn's disease than nonsmokers. Crohn's disease affects between 400,000 and 600,000 people in North America. Prevalence estimates for Northern Europe have ranged from 27-48 per 100,000.Crohn's disease tends to present initially in the teens and twenties, with another peak incidence in the fifties to seventies, although the disease can occur at any age. There is no known pharmaceutical or surgical cure for Crohn's disease. Treatment options are restricted to controlling symptoms, maintaining remission, and preventing relapse.

Ulcerative colitis (Colitis ulcerosa, UC) is a form of inflammatory bowel disease (IBD). Ulcerative colitis is a form of colitis, a disease of the colon (large intestine), that includes characteristic ulcers, or open sores. The main symptom of active disease is usually constant diarrhea mixed with blood, of gradual onset. IBD is often confused with irritable bowel syndrome (IBS), a troublesome, but much less serious, condition. Ulcerative colitis has similarities to Crohn's disease, another form of IBD. Ulcerative colitis is an intermittent disease, with periods of exacerbated symptoms, and periods that are relatively symptom-free. Although the symptoms of ulcerative colitis can sometimes diminish on their own, the disease usually requires treatment to go into remission. Ulcerative colitis has an incidence of 1 to 20 cases per 100,000 individuals per year, and a prevalence of 8 to 246 per 100,000 individuals per year. The disease is more prevalent in northern countries of the world, as well as in northern areas of individual countries or other regions. Rates tend to be higher in more affluent countries, which may indicate the increased prevalence is due to increased rates of diagnosis. Although ulcerative colitis has no known cause, there is a presumed genetic component to susceptibility. The disease may be triggered in a susceptible person by environmental factors. Although dietary modification may reduce the discomfort of a person with the disease, ulcerative colitis is not thought to be caused by dietary factors. Ulcerative colitis is treated as an autoimmune disease. Treatment is with anti-inflammatory drugs, immunosuppression, and biological therapy targeting specific components of the immune response. Colectomy (partial or total removal of the large bowel through surgery) is occasionally necessary if the disease is severe, doesn't respond to treatment, or if significant complications develop. A total proctocolectomy (removal of the entirety of the large bowel) can be curative, but it may be associated with complications.

Metabolic syndrome is a combination of medical disorders that, when occurring together, increase the risk of developing cardiovascular disease and diabetes. Some studies have shown the prevalence in the USA to be an estimated 25% of the population, and prevalence increases with age. Metabolic syndrome is also known as metabolic syndrome X, cardiometabolic syndrome, syndrome X, insulin resistance syndrome, Reaven's syndrome (named for Gerald Reaven), and CHAOS (in Australia). The exact mechanisms of the complex pathways of metabolic syndrome are not yet completely known. The pathophysiology is extremely complex and has been only partially elucidated. Most patients are older, obese, sedentary, and have a degree of insulin resistance. Stress can also be a contributing factor. The most important factors are weight, genetics, endocrine disorders (such as polycystic ovary syndrome in women of reproductive age), aging, and sedentary lifestyle, (i.e., low physical activity and excess caloric intake). There is debate regarding whether obesity or insulin resistance is the cause of the metabolic syndrome or if they are consequences of a more far-reaching metabolic derangement. A number of markers of systemic inflammation, including C-reactive protein, are often increased, as are fibrinogen, interleukin 6, tumor necrosis factor-alpha (TNFα), and others. Some have pointed to a variety of causes, including increased uric acid levels caused by dietary fructose. Central obesity is a key feature of the syndrome, reflecting the fact that the syndrome's prevalence is driven by the strong relationship between waist circumference and increasing adiposity. However, despite the importance of obesity, patients who are of normal weight may also be insulin-resistant and have the syndrome. An estimated 75% of British patients with type 2 diabetes or impaired glucose tolerance (IGT) have metabolic syndrome. The presence of metabolic syndrome in these populations is associated with a higher prevalence of CVD than found in patients with type 2 diabetes or IGT without the syndrome.

Hypoadiponectinemia has been shown to increase insulin resistance, and is considered to be a risk factor for developing metabolic syndrome. The approximate prevalence of the metabolic syndrome in patients with coronary heart disease (CHD) is 50%, with a prevalence of 37% in patients with premature coronary artery disease (age 45), particularly in women. With appropriate cardiac rehabilitation and changes in lifestyle (e.g., nutrition, physical activity, weight reduction, and, in some cases, drugs), the prevalence of the syndrome can be reduced. Lipodystrophic disorders in general are associated with metabolic syndrome. Both genetic (e.g., Berardinelli-Seip congenital lipodystrophy, Dunnigan familial partial lipodystrophy) and acquired (e.g., HIV-related lipodystrophy in patients treated with highly active antiretroviral therapy) forms of lipodystrophy may give rise to severe insulin resistance and many of metabolic syndrome's components.

For many of these inflammatory diseases there is no cure yet. It would be beneficial if the proinflammatory response is suppressed in these inflammatory disease, or to treat or ameliorate the symptoms of inflammatory disease.

It has been surprisingly found that peptides selected from a casein hydrolysate suppresses the proinflammatory response.

### DISCLOSURE OF THE INVENTION

The present disclosure is directed to a nutritional composition for use according to claim 1. Additional features of the nutritional composition are given in claims 2 to 6.

### BRIEF DESCRIPTION OF THE DRAWINGS

Figure 1: Schematic figure of the TIM-1 model: A. stomach compartment; B. pyloric sphincter; C duodenum compartment; D peristaltic valve; E. jejunum compartment; F. peristaltic valve; G. ileum compartment; H. ileo-caecal sphincter; I. stomach secretion; J. duodenum secretion; K jejunum/ileum secretion; L. pre-filter; M. semi permeable membrane; N. water absorption; p pH electrodes; e. level sensors; R temperature sensor; S pressure sensor.
Figure 2 Comparison of peptide profiles after simulated infant stomach digestion observed for a casein hydrolysate of the present disclosure by LC-MS (mlz 230-2000). Peptide profiling is depicted before digestion, after digestion (TIM), and after digestion and further dialysis.
Figure 3. Effect of a casein hydrolysate-containing nutritional composition of the present disclosure on Bacteroides genus level in the microbial community of the fecal fermentation experiment at T=18 following exposure to a digested casein hydrolysate-containing nutritional composition of the present disclosure (thus >500Da) added at low (9.5 mg/ml) or high concentration (19 mg/ml) and determined with 454 pyrosequencing. The y-axis depicts the 10-log value of the number of sequences, which directly correlates to abundance.
Figure 4. Abundance of Bacteroides phylotypes in the microbial community of the fecal fermentation experiment at T=18 following exposure to a digested casein hydrolysate-containing nutritional composition of the present disclosure (thus >500Da) added at low concentration and determined with 454 pyrosequencing. The four main Bacteroides species-like phylotypes (OTU's) are shown. The y-axis depicts the 10-log value of the number of sequences, which directly correlates to abundance.

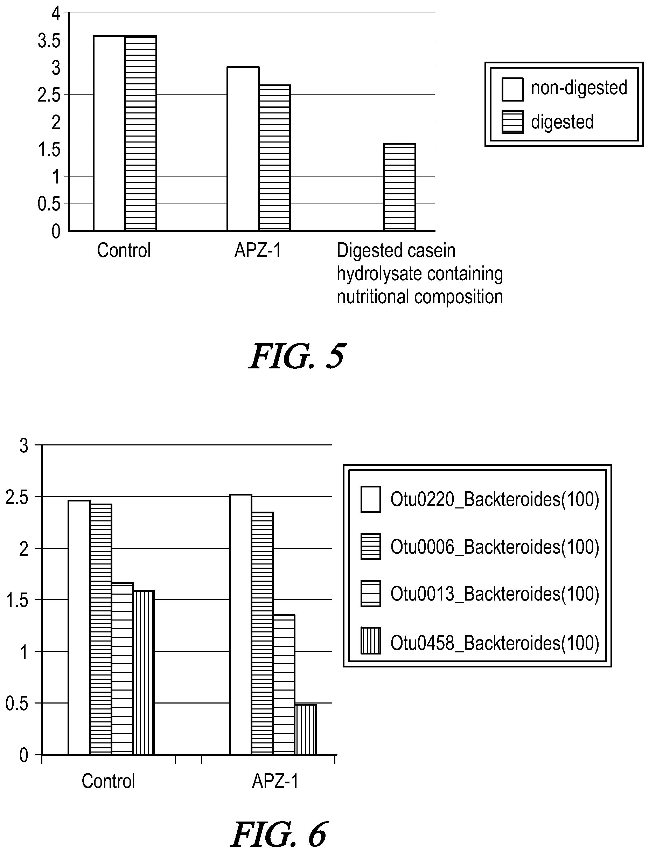

Figure 5. Effect of a casein hydrolysate of the present disclosure (APZ-1) on Bacteroides genus level in the microbial community of the fecal fermentation experiment at T=18 following exposure to non-digested or digested (thus >500Da) casein hydrolysate of the present disclosure added at low concentration and determined with 454 pyrosequencing. The y-axis depicts the 10-log value of the number of sequences correlating to abundance. The casein hydrolysate inhibited Bacteroides abundance, indicating that the effect of the finished product is partly mediated by the hydrolysate/peptide fraction present in the product. A casein hydrolysate-containing nutritional composition of the present disclosure (digested, at low concentration) is included as a reference.
Figure 6. Abundance of Bacteroides phylotypes in the microbial community of the fecal fermentation experiment at T=18 following exposure to digested (thus >500Da) casein hydrolysate of the present disclosure (APZ-1) added at high concentration (19 mg/ml) and determined with 454 pyrosequencing. The y-axis depicts the 10-log value of the number of sequences, which directly correlates to abundance.
Figure 7. Abundance of Bacteroides phylotypes in the microbial community of the fecal fermentation experiment at T=18 following exposure to digested (thus >500Da) casein hydrolysate of the present disclosure (APZ-1) added at low concentration (9.5 mg/ml) and determined with 454 pyrosequencing. The y-axis depicts the 10-log value of the number of sequences, which directly correlates to abundance.
Figure 8. Concentration (microgram/ml) of Glutamic acid at T=0 and T=6 of the fecal fermentation experiments with digested (thus >500Da) casein hydrolysate-containing nutritional composition of the present disclosure finished product at low concentration. Control conditions with MES buffer.
Figure 9. Concentration (microgram/ml) of Glutamic acid at T=0 and T=6 of the fecal fermentation experiments with a non-digested casein hydrolysate of the present disclosure (APZ-1) added at low concentration. Control conditions with MES buffer.
Figure 10. Concentration (microgram/ml) of alpha-amino-n-butyric acid at T=0 and T=6 of the fecal fermentation experiments with digested (thus >500Da) Alimentum Similac and a casein hydrolysate-containing nutritional composition of the present disclosure products added at low concentration. Control conditions with MES buffer.
Figure 11. Concentration (microgram/ml) of alpha-amino-n-butyric acid at T=0 and T=6 of the fecal fermentation experiments with a digested (thus >500Da) casein hydrolysate of the present disclosure (APZ-1) added at low concentration. Control conditions with MES buffer.
Figure 12. Concentration (microgram/ml) of proline at T=0 and T=6 of the fecal fermentation experiments with a digested (thus >500Da) casein hydrolysate-containing nutritional composition of the present disclosure added at low concentration. Control conditions with MES buffer.
Figure 13. Concentration (microgram/ml) of proline at T=0 and T=6 of the fecal fermentation experiments with a digested (thus >500Da) casein hydrolysate of the present disclosure (APZ-1) added at low concentration. Control conditions with MES buffer.
Figure 14. Concentrations (microgram/ml) of butyric acid at T=6 of the fecal fermentation experiments with digested (thus >500Da) Alimentum Similac and a casein hydrolysate-containing formula of the present disclosure added at low (9.5 mg/ml) and high concentrations (19 mg/ml). Control conditions with MES buffer.
Figure 15. Concentrations (microgram/ml) of butyric acid at T=6 of the fecal fermentation experiments with a digested (thus >500Da) casein hydrolysate of the present disclosure (APZ-1) added at low (9.5 mg/ml) and high concentration (19 mg/ml). Control conditions with MES buffer.
Fig. 16 illustrates the pro-inflammatory cytokine secretion of IL12p70 by activated primary human dendritic cells exposed to extensively hydrolyzed casein.
Fig. 17 illustrates the pro-inflammatory cytokine secretion of IL12p70 by activated primary human dendritic cells exposed to a greater than 500 Da fraction of extensively hydrolyzed casein.
Fig. 18 illustrates the pro-inflammatory cytokine secretion of Interferon-gamma by activated human dendritic cells exposed to extensively hydrolyzed casein.
Fig. 19 illustrates the pro-inflammatory cytokine secretion of Interferon-gamma by activated primary human dendritic cells exposed to a greater than 500 Da fraction of extensively hydrolyzed casein.
Fig. 20 illustrates the pro-inflammatory cytokine secretion of Interleukin-8 by activated human dendritic cells exposed to extensively hydrolyzed casein.
Fig. 21 illustrates the pro-inflammatory cytokine secretion of Interleukin-8 by activated primary human dendritic cells exposed to a greater than 500 Da fraction of extensively hydrolyzed casein.
Fig. 22 illustrates the pro-inflammatory cytokine secretion of Tumor Necrosis Factor-alpha by activated human dendritic cells exposed to extensively hydrolyzed casein.
Fig. 23 illustrates the pro-inflammatory cytokine secretion of Tumor Necrosis Factor-alpha by activated primary human dendritic cells exposed to a greater than 500 Da fraction of extensively hydrolyzed casein.
Fig. 24 illustrates the pro-inflammatory cytokine secretion of Interleukin-6 by activated human dendritic cells exposed to extensively hydrolyzed casein.
Fig. 25 illustrates the pro-inflammatory cytokine secretion of Interleukin-6 by activated primary human dendritic cells exposed to a greater than 500 Da fraction of extensively hydrolyzed casein.
Fig. 26 illustrates the pro-inflammatory cytokine secretion of Interleukin-1β by activated human dendritic cells exposed to extensively hydrolyzed casein.
Fig. 27 illustrates the pro-inflammatory cytokine secretion of Interleukin-1β by activated human dendritic cells exposed to a greater than 500 Da fraction of extensively hydrolyzed casein.
Fig. 28 shows the secretion of Tumor Necrosis Factor-alpha of activated primary human macrophages when exposed to extensively hydrolyzed casein.
Fig. 29 illustrates the secretion of Tumor Necrosis Factor-alpha of activated primary human macrophages when exposed to a greater than 500 Da fraction of extensively hydrolyzed casein.
Fig. 30 illustrates the secretion of Interleukin-1 (IL1) in the ileum of NOD mice, BALB/C mice and NOD mice fed an extensively hydrolyzed casein diet.
Fig. 31 illustrates the secretion of Interleukin-18 (IL18) in the ileum of NOD mice, BALB/C mice and NOD mice fed an extensively hydrolyzed casein diet.
Fig. 32 illustrates the secretion of Interleukin-6 (IL6) in the ileum of NOD mice, BALB/C mice and NOD mice fed an extensively hydrolyzed casein diet.
Fig. 33 illustrates the secretion of Interleukin-17 (IL17) in the ileum of NOD mice, BALB/C mice and NOD mice fed an extensively hydrolyzed casein diet.
Fig. 34 illustrates the secretion of IFN-gamma of gut-draining lymph node T cells of NOD mice, BALB/C mice and NOD mice fed an extensively hydrolyzed casein diet.
Fig. 35 illustrates the secretion of Interleukin-4 of gut-draining lymph node T cells of NOD mice, BALB/C mice and NOD mice fed an extensively hydrolyzed casein diet.
Fig.36 illustrates the secretion of Interleukin-17 of gut-draining lymph node T cells of NOD mice, BALB/C mice and NOD mice fed an extensively hydrolyzed casein diet.
Fig. 37 illustrates the pro-inflammatory cytokine secretion of Interleukin-12p40 from mouse macrophages exposed to extensively hydrolyzed casein, non-sterile filtered extensively hydrolyzed casein, and a greater than 500 Da fraction of extensively hydrolyzed casein.
Fig. 38 illustrates the pro-inflammatory cytokine secretion of Interleukin-1β from mouse macrophages exposed to extensively hydrolyzed casein, non-sterile filtered extensively hydrolyzed casein, and a greater than 500 Da fraction of extensively hydrolyzed casein.
Fig. 39 illustrates the pro-inflammatory cytokine secretion of Interleukin-6 from mouse macrophages exposed to extensively hydrolyzed casein, non-sterile filtered extensively hydrolyzed casein, and a greater than 500 Da fraction of extensively hydrolyzed casein.

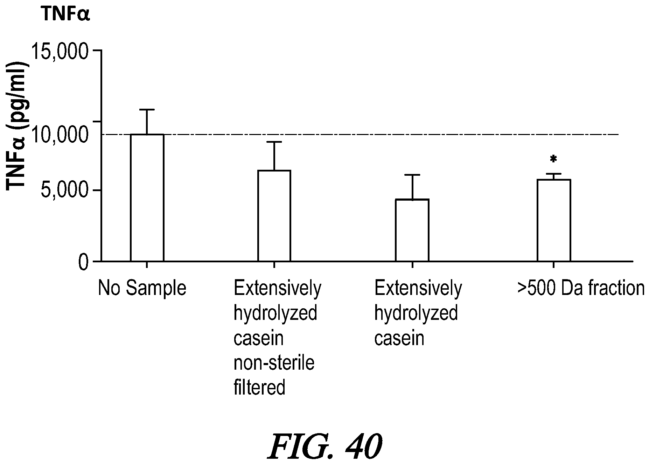

Fig. 40 illustrates the pro-inflammatory cytokine secretion of Tumor necrosis factor-alpha from mouse macrophages exposed to extensively hydrolyzed casein, non-sterile filtered extensively hydrolyzed casein, and a greater than 500 Da fraction of extensively hydrolyzed casein.
Fig. 41 illustrates the pro-inflammatory cytokine secretion of Interleukin-12p40, Interleukin-1β, Interleukin-6, and Tumor Necrosis Factor-alpha from mouse macrophages exposed to extensively hydrolyzed casein, non-sterile filtered extensively hydrolyzed casein, and a greater than 500 Da fraction of extensively hydrolyzed casein.

### BEST MODE FOR CARRYING OUT THE INVENTION

The term "nutritional composition" as used herein describes a solid or liquid formulation which can therefore be eaten or drunk by a human subject for nutrition. The nutritional composition of the disclosure preferably has a nutritional value of at least 1, more preferred at least 10 and even more preferred 50 kcal(kilo calorie)/100ml for liquid formulations and preferably at least 1, more preferred at least 10, even more preferred at least 50, such as at least 100, and most preferred at least 300 kcal/100g for dry food formulations. In the embodiment of the disclosure the nutritional formulation of the disclosure has a nutritional value of at least 50-200 kcal/100ml for liquid formulations and at least 300-600 kcal/100g for dry food formulations. A nutritional composition is distinguished from a vaccine. In contrast to a vaccine, a nutritional composition does not comprise any of adjuvants (unless as contaminations), activated or inactivated viral compounds (unless as contaminations), activated or inactivated bacterial compounds (unless as contaminations), and pathogenic compounds (unless as contaminations). The term "supplement" as used
herein relates to a nutritional supplement which is a concentrated source of nutrient or alternatively other substances with a nutritional or physiological effect whose purpose is to supplement the normal diet.

In addition to the above recited ingredients further ingredients may be selected from lipids, minerals, carbohydrates, amino acids, amino acid chelates, anabolic nutrients, vitamins, antioxidants, probiotic bacterial strain and lipotropic agents in order to provide an optimal sustained energy and anabolic nutritional formulation. The nutritional composition may be a nutritional supplement or may provide complete nutrition. Preferably the nutritional composition is in the form of a dry food concentrate. The nutritional composition of the disclosure provides a human subject with increasing preference with at least 5%, at least 10%, at least 25%, at least 50%, at least 75% or at least 90% of the daily calorie requirement of a human subject. The person skilled in the art is well aware that the daily calorie requirement is dependent on the gender, height and age of a human subject. For example, a 30 year old male of 80kg body weight and 180cm height has a daily calorie requirement of around 2900 cal (calories) to maintain his body weight whereas a 30 year old female of 55kg body weight and 165cm height has a daily calorie requirement of around 2100 cal to maintain her body weight. In the embodiment, the nutritional formulation of the present disclosure is an infant or a nutritional product for infants or juvenile.

The term "peptide" as used herein describes linear molecular chains of amino acids, including single chain molecules or their fragments. A peptide in accordance with the disclosure contains with increasing preference about 2 to 100 amino acids, about 5 to 50 amino acids, or about 5 to 40 amino acids. Peptides may further form oligomers consisting of at least two identical or different molecules. The corresponding higher order structures of such multimers are, correspondingly, termed homo- or heterodimers, homo- or heterotrimers etc. Furthermore, peptidomimetics of such peptides where amino acid(s) and/or peptide bond(s) have been replaced by functional analogs are also encompassed by the term "peptide". Such functional analogues include all known amino acids other than the 20 gene- encoded amino acids, such as selenocysteine. The term "peptide" also refers to naturally modified peptides where the modification is effected e.g. by glycosylation, acetylation, phosphorylation and similar modifications which are well known in the art. A peptide has to be distinguished from a protein in the present disclosure. A protein in accordance with the present disclosure describes an organic compound made of amino acids arranged in a linear chain and folded into a globular form. Furthermore, a protein in accordance with the present disclosure describes a chain of amino acids of more than 100 amino acids. Peptides may, e.g., be produced recombinantly, (semi-) synthetically, or obtained from natural sources such as after hydrolysation of proteins, all according to methods known in the art.

The term "casein hydrolysate" as used herein defines a formula which comprises peptides derived from hydrolyzed cow's casein milk proteins.. In this regard, a hydrolyzed protein is a protein that has been broken down into peptides and/or component amino acids. While there are many means of achieving protein hydrolysis, two of the most common means are prolonged boiling in a strong acid or strong base or using an enzyme such as the pancreatic protease enzyme to stimulate the naturally-occurring hydrolytic process. Hydrolysis of proteins derived from milk is preferably achieved using an enzyme or a mixture of enzyme. A casein cow milk hydrolysate can comprise peptides derived from milk, wherein the proteins of said milk have been hydrolyzed to various degrees. Accordingly, one can distinguish between a partially hydrolyzed cow's milk peptide-containing hydrolysate and an extensively hydrolyzed cow's milk peptide- containing hydrolysate. In this regard, a partially hydrolyzed cow's milk peptide- containing hydrolysate comprises more than 20% of intact cow's milk protein whereas an extensively hydrolyzed cow's milk peptide-containing hydrolysate comprises less than 1% of peptides having a size of greater than 1.5kD. Furthermore, an extensively hydrolyzed cow's milk peptide-containing hydrolysate is preferably hypoallergenic.

A nonlimiting example of a method of hydrolysis is disclosed herein.This method may be used to obtain the protein hydrolysate and peptides of the present disclosure. The proteins are hydrolyzed using a proteolytic enzyme, Protease N. Protease N "Amano" is commercially available from Amano Enzyme U.S.A. Co., Ltd., Elgin, III. Protease N is a proteolytic enzyme preparation that is derived from the bacterial species Bacillus subtilis. The protease powder is specified as "not less than 150,000 units/g", meaning that one unit of Protease N is the amount of enzyme which produces an amino acid equivalent to 100 micrograms of tyrosine for 60 minutes at a pH of 7.0. To produce the infant formula of the present invention, Protease N can be used at levels of about 0.5% to about 1.0% by weight of the total protein being hydrolyzed.

The protein hydrolysis by Protease N is typically conducted at a temperature of about 50° C. to about 60° C. The hydrolysis occurs for a period of time so as to obtain a degree of hydrolysis between about 4% and 10%.Hydrolysis occurs for a period of time so as to obtain a degree of hydrolysis between about 6% and 9%. Alternatively, hydrolysis occurs for a period of time so as to obtain a degree of hydrolysis of about 7.5%. This level of hydrolysis may take between about one half hour to about 3 hours.

A constant pH should be maintained during hydrolysis. The pH is adjusted to and maintained between about 6.5 and 8. In particular, the pH is maintained at about 7.0.

In order to maintain the optimal pH of the solution of whey protein, casein, water and Protease N, a caustic solution of sodium hydroxide and/or potassium hydroxide can be used to adjust the pH during hydrolysis. If sodium hydroxide is used to adjust the pH, the amount of sodium hydroxide added to the solution should be controlled to the level that it comprises less than about 0.3% of the total solid in the finished protein hydrolysate. A 10% potassium hydroxide solution can also be used to adjust the pH of the solution to the desired value, either before the enzyme is added or during the hydrolysis process in order to maintain the optimal pH.

The amount of caustic solution added to the solution during the protein hydrolysis can be controlled by a pH-stat or by adding the caustic solution continuously and proportionally. The hydrolysate can be manufactured by standard batch processes or by continuous processes.

To better ensure the consistent quality of the protein partial hydrolysate, the hydrolysate is subjected to enzyme deactivation to end the hydrolysis process. The enzyme deactivation step may consist include at heat treatment at a temperature of about 82° C. for about 10 minutes. Alternatively, the enzyme can be deactivated by heating the solution to a temperature of about 92° C. for about 5 seconds. After enzyme deactivation is complete, the hydrolysate can be stored in a liquid state at a temperature lower than 10° C.

A method for producing a protein partial hydrolysate includes the following procedure. Initially, 60.3 kg non-milk solids (milk powder) and 37.4 kg whey protein concentrate (60%) were intermixed in a tank containing water at 54° C. The slurry had a total solids content of between 20% and 23%. The pH of the slurry was then measured. Sodium and potassium hydroxide were added to the slurry to adjust the pH of the slurry to 7.0. After adjusting the pH, 0.5 kg of Amano N enzyme was added to the slurry. Following the addition of Amano N to the slurry, the pH was continuously adjusted to a pH of 7.0 using sodium hydroxide and potassium hydroxide. The total amount of sodium hydroxide added to the slurry was 0.3 kg. The total amount of potassium hydroxide added to the slurry was 1.5 kg.

The hydrolysis was permitted to occur for 90 minutes, the time starting with the addition of Amano N enzyme to the slurry. At the end of 90 minutes, the slurry was heat treated to inactivate the enzyme. The heat treatment consisted of raising the temperature of the slurry to 82° C. for 10 minutes. The degree of hydrolysis obtained in this example was between 6% and 9%. The slurry was then cooled and spray dried to obtain a powdered hydrolysate.

A non-limiting method of determining the molecular weight distribution of the hydrolysate peptides includes. Size exclusion chromatography (SEC) to determine the molecular weight distribution of the hydrolysate peptides created by the presently-described hydrolysis process. Specifically, a sufficient amount of the powdered infant formula was weighed out to provide 0.5 grams of protein into a 50 ml conical centrifuge tube. Water was added to bring the tube to a volume of 45 ml. The mixture was placed in a Sarstedt D-5223 Mixer and mixed for one hour. After mixing, a 1% protein solution was created by adding another 5 ml of water to the tube. A stock standard was prepared and mixed for one hour as well. Separately, 14.91 grams potassium chloride (KCI) was added to a 1000 ml beaker. The KCI was dissolved by adding 700 ml of water to the beaker. 250 ml acetonitrile and 1.0 ml trifloroacetic acid were then added to the KCI solution (eluent). The pH was adjusted to 3.0 using a 0.2M K2HPO4 solution. An HPCL reagent bottle was filled and the bottle was washed with eluent, reserving about 50 ml for dilution of samples and standards. The Hitachi L-6200 A Intelligent Pump lines were purged with eluent and the columns were equilibrated with eluent for one hour. After the samples were mixed for one hour, 5.0 ml of each sample was pipetted into glass screw-cap tubes. 5.0 ml Dichloromethane was also pipetted into each tube. The tubes were capped and mixed by inversion four times. The samples were then centrifuged for five minutes at 200xg. While the samples were in the centrifuge, the stock standards 1-5 were diluted with eluent (800 ul+3200 ul). Approximately 1 ml of each standard was pipetted into each of two autosampler vials and capped. The upper (aqueous) layer of the centrifuged samples 1-10 were diluted with eluent (100 ul+900 ul). The vials were loaded into the autosampler tray as follows: blank, standard, samples and second standard. The tray was placed in the Hitachi autosampler. The total number of vials to be run were entered into the autosampler program using the keys on the front of the autosampler and the samples were run. The results indicated the molecular weight profile of the protein.

The term "peptide derived from cow's milk" as used herein defines a peptide which has an amino acid sequence which is a partial amino acid sequence of a cow's milk protein. Such peptides may be obtained as outlined above by hydrolysis or may be synthesized in vitro by methods known to the skilled person and described in the examples of the disclosure.

The term "peptide-containing fraction of the hydrolysate" refers to a mixture of peptides comprising at least 2, preferably at least 5, more preferably at least 10 and most preferably at least 20 which have been isolated from the hydrolysate of the disclosure by filtration techniques which are known to the skilled person. Furthermore, techniques for the isolation of peptides from the hydrolysate of the disclosure are described herein below.

The term "child" or the term "juvenile" is used herein in accordance with the definitions provided in the art. Thus, the term "child" means a human subject between the stages of birth and the age of about 10 and the term "juvenile" means a human subject between the age of about 10 and puberty (before sexual maturity).

The term "adult" is used herein in accordance with the definitions provided in the art. Thus, this term means a human subject after puberty (after sexual maturity).

The term "cow's milk allergy" describes a food allergy, i.e. an immune adverse reaction to one or more of the proteins contained in cow's milk in a human subject. The principal symptoms are gastrointestinal, dermatological and respiratory symptoms. These can translate into skin rashes, hives, vomiting, diarrhea, constipation and distress. The clinical spectrum extends to diverse disorders: anaphylactic reactions, atopic dermatitis, wheeze, infantile colic, gastro esophageal reflux disease (GERD), esophagitis, colitis gastroenteritis, headache/migraine and constipation.

In the embodiment of the disclosure the inflammatory disease is selected from the group consisting of type 1 diabetes, Crohn's disease, Ulcerative colitis, Metabolic syndrome.]

The present inventors have surprisingly found that a casein hydrolysate has a beneficial effect on the suppression of pro-inflammatory response. It was also found that especially an extensively hydrolyzed cow's milk peptide-containing hydrolysate had positive effects on the on the risk factors of inflammatory disease and especially type 1 diabetes. Suitable hydrolysates are casein hydrolysates. It was furthermore found that dialysis of the hydrolysate with a cut-off of 500 Da renders a hydrolysate fraction that has even better effect on the suppression of proinflammatory response. Accordingly, in the embodiments, the hydrolysate consists of peptides with a molecular weight of more than 500 Da.

The following peptides have been identified in the hydrolysate and may have a beneficial effect on an inflammatory response:

**Table 1: identified peptide in the hydrolysate:**

| | |
|---|---|
| SEQ ID NO: 1* | IPNPIG |
| SEQ ID NO: 2 | IGSESTEDQ |
| SEQ ID NO 3: | DKTEIPT |
| SEQ ID NO: 4 | IVPN |
| SEQ ID NO: 5 | LEDSPE |
| SEQ ID NO: 6 | NQEQPI |
| SEQ ID NO: 7 | NVPGE |
| SEQ ID NO: 8 | PFPGPI |
| SEQ ID NO: 9 | TEDEL |
| SEQ ID NO: 10 | VPSE |
| SEQ ID NO: 11 | YPFPGP |
| SEQ ID NO: 12 | YPSGA |
| SEQ ID NO 13 | FPGPIP |
| SEQ ID NO: 14 | MHQPHQPLPPT |
| SEQ ID NO: 15 | YPFPGPIPN |
| SEQ ID NO: 16 | DMEST |
| SEQ ID NO: 17 | FPGPIPN |
| SEQ ID NO: 18 | IPNPI |
| SEQ ID NO: 19 | MESTEV |
| SEQ ID NO: 20 | PGPIPN |
| SEQ ID NO: 21 | PHQPLPPT |
| SEQ ID NO: 22 | PNPI |
| SEQ ID NO: 23 | SKDIGSE |
| SEQ ID NO: 24 | YPFPGPIP |
| SEQ ID NO: 25 | AINPSKEN |
| SEQ ID NO: 26 | APFPE |
| SEQ ID NO: 27 | DIGSES |
| SEQ ID NO: 28 | DMPI |
| SEQ ID NO: 29 | DVPS |
| SEQ ID NO: 30 | EDI |
| SEQ ID NO: 31 | ELF |
| SEQ ID NO: 32 | EMP |
| SEQ ID NO: 33 | ETAPVPL |
| SEQ ID NO: 34 | GPFP |
| SEQ ID NO: 35 | GPIV |
| SEQ ID NO: 36 | IGSSSEES |
| SEQ ID NO: 37 | IGSSSEESA |
| SEQ ID NO: 38 | INPSKE |
| SEQ ID NO: 39 | IPPLTQTPV |
| SEQ ID NO: 40 | ITAP |
| SEQ ID NO: 41 | KHQGLPQ |
| SEQ ID NO: 42 | LDVTP |
| SEQ ID NO: 43 | LPLPL |
| SEQ ID NO: 44 | NAVPI |
| SEQ ID NO: 45 | NEVEA |
| SEQ ID NO: 46 | NLL |
| SEQ ID NO: 47 | PITPT |
| SEQ ID NO: 48 | PNSLPQ |
| SEQ ID NO: 49 | PQLEIVPN |
| SEQ ID NO: 50 | PQNIPPL |
| SEQ ID NO: 51 | PVLGPV |
| SEQ ID NO: 52 | PVPQ |
| SEQ ID NO: 53 | PVVVP |
| SEQ ID NO: 54 | PVVVPP |
| SEQ ID NO: 55 | SIGSSSEESAE |
| SEQ ID NO: 56 | SISSSEE |
| SEQ ID NO: 57 | SISSSEEIVPN |
| SEQ ID NO: 58 | SPPEIN |
| SEQ ID NO: 59 | SPPEINT |
| SEQ ID NO:60 | TDAPSFS |
| SEQ ID NO: 61 | VATEEV |
| SEQ ID NO: 62 | VLPVP |
| SEQ ID NO: 63 | VPGE |
| SEQ ID NO: 64 | VPGEIV |
| SEQ ID NO: 65 | VPITPT |
| SEQ ID NO: 66 | VVPPFLQPE |
| SEQ ID NO: 67 | VVVPP |
| SEQ ID NO: 68 | YPVEP |

In the embodiment of the present disclosure the hydrolysate comprises a peptide with SEQ ID NO: 1 and at least two peptides selected from the group consisting of SEQ ID NO: 2-68, preferably at least 3 peptides, preferably at least 4 peptides, preferably at least 5 peptides selected from the group consisting of SEQ ID NO: 2-68.

The nutritional composition comprises an oligosaccharide.

The nutritional composition comprising peptides selected from a casein hydrolysate.

The disclosure is now exemplified by the following non-limiting examples.

### Examples:

### Example 1: Digestion model set up.

All formulations (finished formulas and hydrolysates) were investigated in the TIM-1 system (TNO, Zeist, The Netherlands) simulating the average conditions in the stomach and small intestine of young children (0-0.5 year of age). These conditions included especially the dynamics of gastric emptying, the gastric and the intestinal pH values and the compositions and activities of the oral, gastric and intestinal secretion fluids such as electrolytes, digestive enzymes and bile. Digested and dialysed (to remove mono- and di-saccharides) materials of the finished products as well as undigested and digested and dialysed materials of the hydrolysates were used for infant fecal fermentation experiments to study possible effects on protein/peptide digestion by the colonic microbial community and to study the effects of these components on gut microbiota composition and metabolism. From the fecal fermentations, samples were taken at different time points and were subjected to different types of analyses.

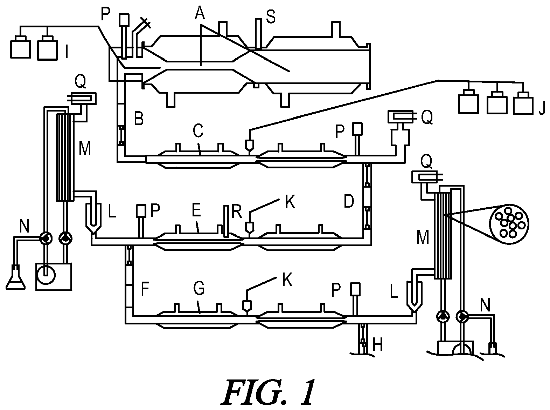

Figure 1 shows the setup. It shows a schematic figure of the TIM-1 model: A. stomach compartment; B. pyloric sphincter; C duodenum compartment; D peristaltic valve; E. jejunum compartment; F. peristaltic valve; G. ileum compartment; H. ileo-caecal sphincter; I. stomach secretion; J. duodenum secretion; Kjejunum/ileum secretion; L. pre-filter; M. semi permeable membrane; N. water absorption; p pH electrodes; e. level sensors; R temperature sensor; S pressure sensor. Peptide profiles after stomach digestion:

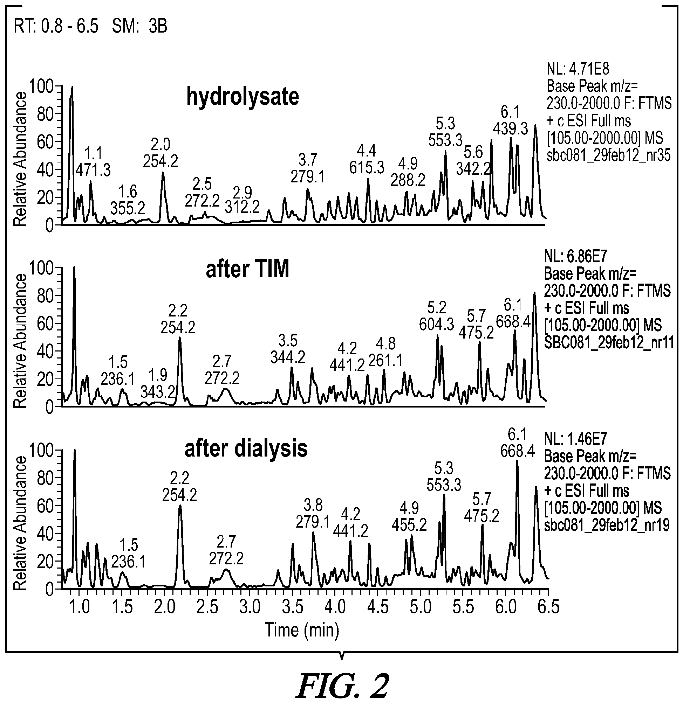

Figure 2 shows a comparison of peptide profiles after simulated infant stomach digestion observed for the a casein hydrolysate of the present disclosure by LC-MS (mlz 230-2000). Peptide profiling is depicted before digestion, after digestion (TIM), and after digestion and further dialysis. The figures show that the peptide profile of the hydrolysate hardly changed during simulated stomach digestion.

### Microbiota composition and metabolism

The below findings were generated with finished products or hydrolysates after dialyses, thus generating a product with peptides having a molecular weight of more than 500 Da.

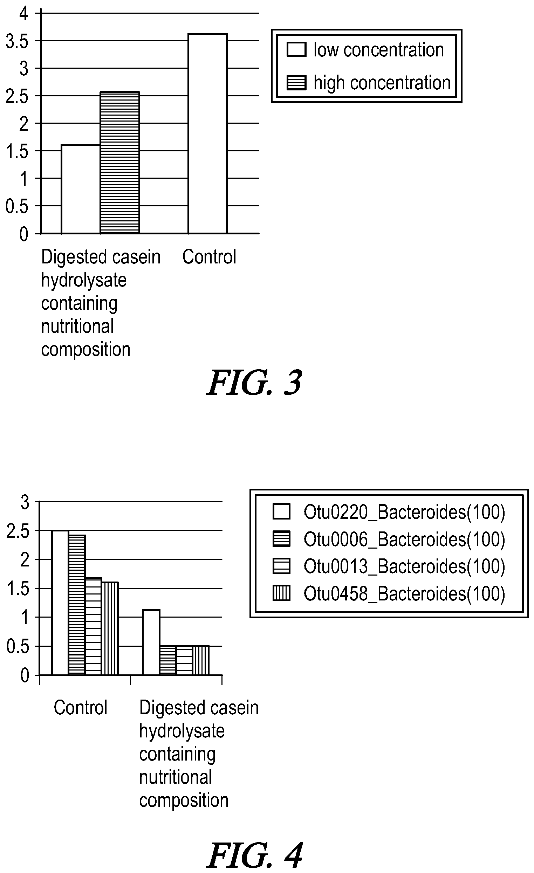

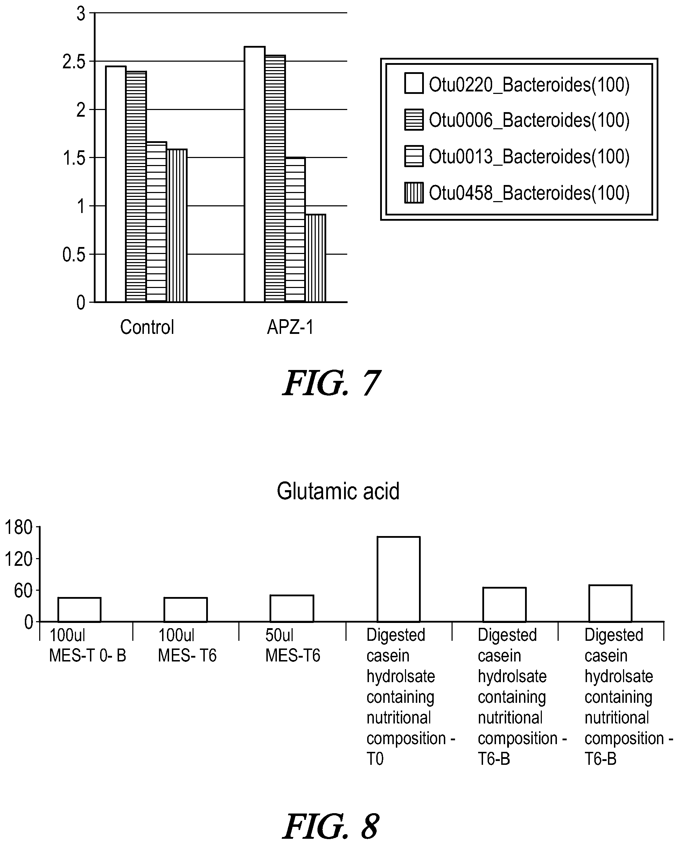

The below findings were generated with finished products or hydrolysates after dialyses, thus generating a product with peptides having a molecular weight of more than 500 Da. Figure 3 shows the effect of a casein hydrolysate-containing nutritional composition of the present disclosure on Bacteroides genus level in the microbial community of the fecal fermentation experiment at T=18 following exposure to a digested casein hydrolysate-containing nutritional composition of the present disclosure (thus >500Da) added at low or high concentration and determined with 454 pyrosequencing. The y-axis depicts the 10-log value of the number of sequences, which directly correlates to abundance. A casein hydrolysate-containing nutritional composition of the present disclosure clearly inhibited Bacteroides abundance, which may have been mediated by both carbohydrates as well as peptides present in the product. Figure 4. Shows the abundance of Bacteroides phylotypes in the microbial community of the fecal fermentation experiment at T=18 following exposure to a digested casein hydrolysate-containing nutritional composition of the present disclosure (thus >500Da) added at low concentration and determined with 454 pyrosequencing. The four main Bacteroides species-like phylotypes (OTU's) are shown. The y-axis depicts the 10-log value of the number of sequences, which directly correlates to abundance. The casein hydrolysate-containing nutritional composition clearly inhibited Bacteroides abundance, which may have been mediated by both carbohydrates as well as peptides present in the product. Figure 5 shows the effect of a casein hydrolysate of the present disclosure (APZ-1) on Bacteroides genus level in the microbial community of the fecal fermentation experiment at T=18 following exposure to non-digested or a digested (thus >500Da) casein hydrolysate of the present disclosure added at low concentration and determined with 454 pyrosequencing. The y-axis depicts the 10-log value of the number of sequences correlating to abundance. A casein hydrolysate of the present disclosure inhibited Bacteroides abundance, indicating that the effect of the finished product is partly mediated by the hydrolysate/peptide fraction present in the product. A casein hydrolysate-containing nutritional composition of the present disclosure (digested, at low concentration) is included as a reference. Figure 6 & 7 shows the abundance of Bacteroides phylotypes in the microbial community of the fecal fermentation experiment at T=18 following exposure to a digested (thus >500Da) casein hydrolysate of the present disclosure (APZ-1) added at high (figure 6) (19 mg/ml) or low (figure 7) concentration (9.5 mg/ml) and determined with 454 pyrosequencing. The y-axis depicts the 10-log value of the number of sequences, which directly correlates to abundance. A casein hydrolysate of the present disclosure clearly inhibited Bacteroides abundance, indicating that the effect of the finished product is partly mediated by the hydrolysate/peptide fraction present in the product. Interestingly, the hydrolysate mainly affects OTU 013 and 458, related to B. stercoris/uniformis, whereas little to no effect was observed on OTUs 006 (B. thetaiotaomicron group) and 0220 (B. fragilis group). In literature (Giongo et al, 2010), increased abundance of B. uniformis has been observed in the microbiota of children developing T1D and B. fragilis in control children, implying that the effects of the hydrolysate on these groups may be beneficial against the development of T1D.

In the in vitro model of infant fecal sample fermentation there was no obvious effect of the casein hydrolysate-containing nutritional composition of the present disclosure or casein hydrolysate of the present disclosure on Bifidobacteria composition. The abundance of Bacteroides species was severely impacted following exposure of the faecal community to various commercial products including a casein hydrolysate-containing nutritional composition of the present disclosure, and this may also involve carbohydrate fermentation. Exposure of the faecal ecosystem to the hydrolysates impacted the Bacteroides group, as determined by qPCR. There was a significant growth inhibiting effect of a casein hydrolysate of the present disclosure on Bacteroides stercoris/uniformis group. There was little to no effect on B. thetaiotamicron or B. fragilis group. Note that certain Bacteroides species, ovatus, fragilis, vulgates, uniformis may have been associated with auto-immune or chronic inflammatory diseases. In this respect reduction of Bacteroides species could imply a potential health benefit.

### Amino acids and short chain fatty acids

Figure 8 shows the concentration of Glutamic acid at T=0 and T=6 of the fecal fermentation experiments with a digested (thus >500Da) casein hydrolysate-containing nutritional composition of the present disclosure at low concentration. Control conditions with MES buffer. Glutamate concentration (microgram/ml) as added with finished product decreased during fermentation. This could indicate a beneficial effect since elevated levels of glutamate in plasma are associated with T1D onset (Oresic et al, 2008).

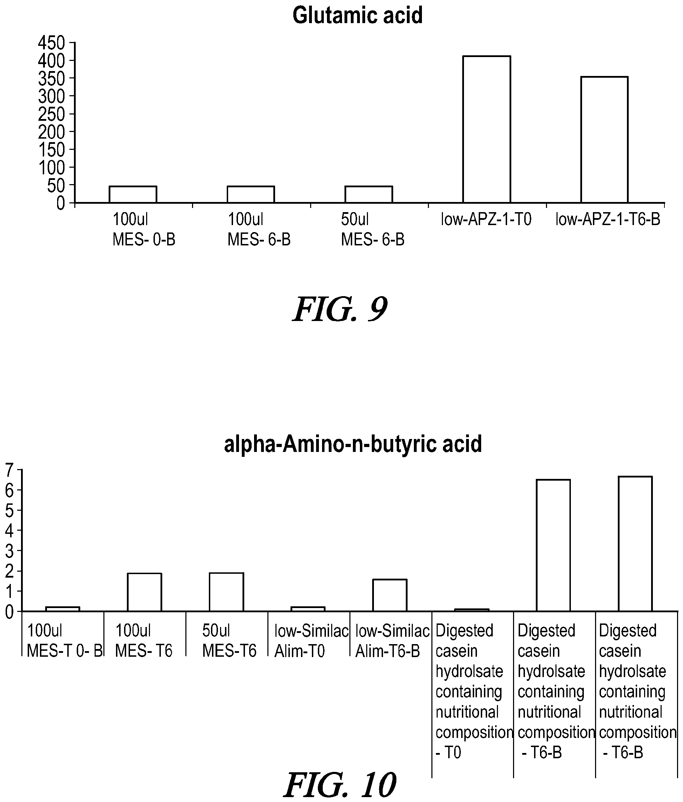

Figure 9 shows the concentration of Glutamic acid at T=0 and T=6 of the fecal fermentation experiments with a non-digested casein hydrolysate of the present disclosure (APZ-1) added at low concentration. Control conditions with MES buffer. The concentration(microgram/ml) of glutamate as added with the hydrolysate slightly decreased during the fermentation, indicating that the effects of a casein hydrolysate-containing nutritional composition of the present disclosure on glutamate levels might partly be mediated by the hydrolysate/peptide fraction present in the product.

Production of glutamate decreased after fermentation of a casein hydrolysate-containing nutritional composition of the present disclosure, a casein hydrolysate and amino acid containing nutritional composition of the present disclosure and Similac, accompanied by increased proline and threonine as compared to other products.

Figure 10 shows concentration of alpha-amino-n-butyric acid (microgram/ml) at T=0 and T=6 of the fecal fermentation experiments with digested (thus >500Da), product Alimentum Similac and casein hydrolysate-containing nutritional compositions of the present disclosure added at low concentration. Control conditions with MES buffer. The casein hydrolysate-containing nutritional compositions of the present disclosure stimulated the production of alpha-amino-n-butyric acid during fermentation to a much larger extent than Similac.

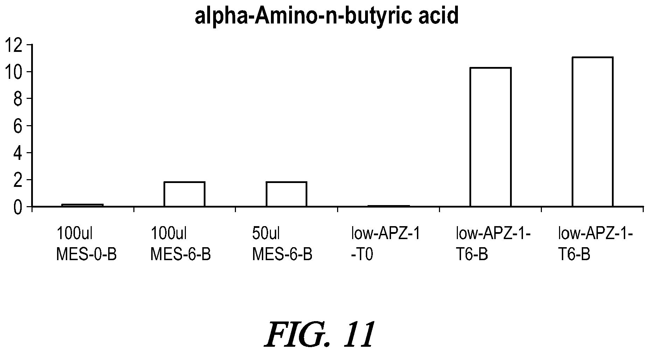

Figure 11 shows the concentration of alpha-amino-n-butyric acid (microgram/ml) at T=0 and T=6 of the fecal fermentation experiments with digested (thus >500Da) casein hydrolysate of the present disclosure (APZ-1) added at low concentration. Control conditions with MES buffer. These results strongly indicate the hydrolysate/peptide fraction to contribute to the effects of the casein hydrolysate-containing nutritional compositions.

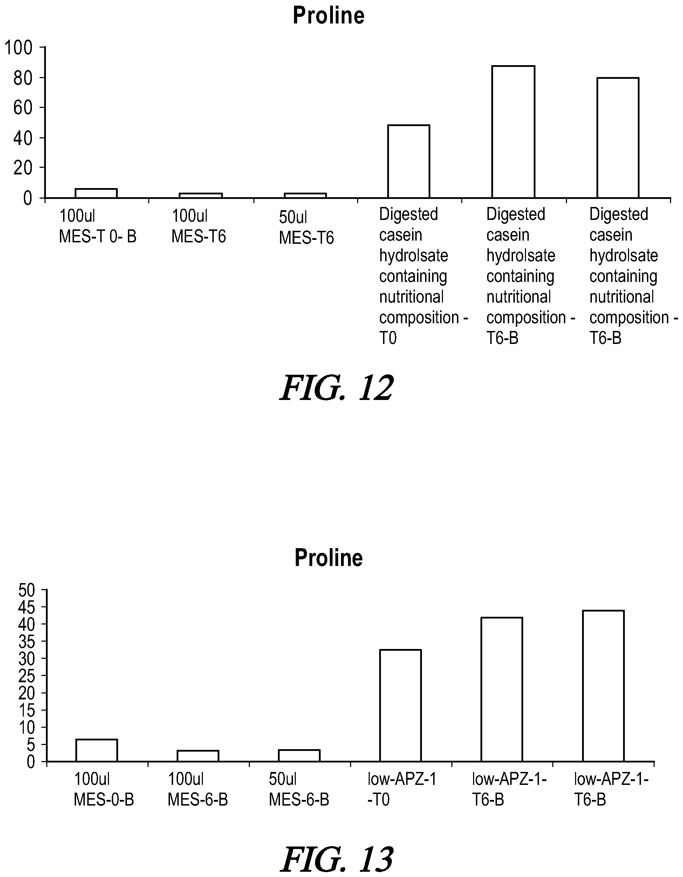

Figure 12 shows the concentration of proline (microgram/ml) at T=0 and T=6 of the fecal fermentation experiments with a digested (thus >500Da) a casein hydrolysate-containing nutritional composition of the present disclosure added at low concentration. Control conditions with MES buffer. The casein hydrolysate-containing nutritional composition of the present disclosure stimulated proline production during the fermentation.

Figure 13 shows the concentration of proline (microgram/ml) at T=0 and T=6 of the fecal fermentation experiments with a digested (thus >500Da) a casein hydrolysate of the present disclosure (APZ-1) added at low concentration. Control conditions with MES buffer. These results strongly suggest the hydrolysate/peptide fraction to be involved in the proline stimulating effect of casein hydrolysate-containing nutritional compositions of the present disclosure.

Fermentation of a casein hydrolysate-containing nutritional composition of the present disclosure and a casein hydrolysate and amino acid containing nutritional composition of the present disclosure led to stronger increase in hydroxyproline than other products, whereas lysine was less depleted.

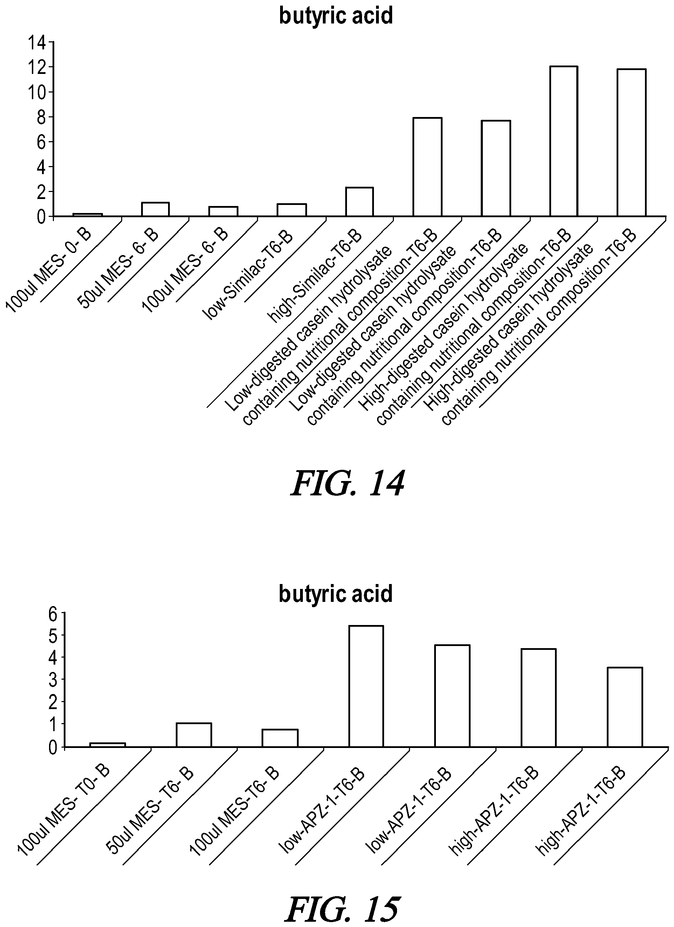

Figure 14 shows the concentrations (microgram/ml) of butyric acid at T=6 of the fecal fermentation experiments with digested (thus >500Da) Alimentum Similac and casein hydrolysate-containing nutritional compositions of the present disclosure added at low (9.5 mg/ml) and high concentrations (19 mg/ml). Control conditions with MES buffer. The casein hydrolysate-containing nutritional compositions of the present disclosure led to an increased production of butyrate as compared to Similac.

Figure 15 shows the concentrations (microgram/ml) of butyric acid at T=6 of the fecal fermentation experiments with digested (thus >500Da) a casein hydrolysate of the present disclosure (APZ-1) added at low (9.5 mg/ml) and high concentration (19 mg/ml). Control conditions with MES buffer. These results indicate the hydrolysate/peptides present in the finished product to contribute to the effects on butyrate production.

The highest production of butyrate was observed in the fermentations with a casein hydrolysate-containing nutritional compositions of the present disclosure as compared to other products, also reflected in the highest ratio of butyrate:(acetate+propionate).

In the fermentation experiments casein hydrolysate-containing nutritional compositions of the present disclosure was found to be superior to other products in stimulation a variety of aminoacids (glutamate, threonine) and short chain fatty acids, esp. butyric acid.

### Stimulation of CCK

A casein hydrolysate of the present disclosure hydrolysate, as compared with a partial hydrolysate (e.g., Gentlease) or free amino acids was found to stimulate CCK release from STC-1 (intestinal endocrine cell line). Moreover, specific peptides from a casein hydrolysate of the present disclosure may stimulate CCK release form STC-1 cells (DISLLDAQSAPLR, ALPMHIR, GPVRGPFP) These findings may be translated into a potential satiety and gastrointestinal motility supporting effect. Animal model (NOD mouse)

A casein hydrolysate-containing nutritional composition of the present disclosure and a casein hydrolysate of the present disclosure reduce the incidence of T1D in experimental animal models (NOD mouse and DP-BB rat models respectively).

### EXAMPLE 2:

Example 2 is directed to the inhibition of pro-inflammatory cytokines in primary human dendritic cell assays by extensively hydrolyzed casein and a greater than 500 Da fraction thereof. Specifically, the pro-inflammatory cytokines inhibited include, Interleukin-12p70 ("Il12p70"), Interferon-gamma (IFNý), Interleukin-8 ("IL8"), Tumor Necrosis Factor-alpha ("TNFa"), Interleukin-6 ("IL6"), and Interleukin-lbeta ("IL1β"). Isolated CD14+ monocytes were suspended in RPMI medium and supplemented with 10% heat-inactivated fetal bovine serum ("FBS"), penicillin/streptomycin, 500 IU/ml recombinant Interleukin-4 ("rhIL-4"), and 800 IU/ml rhGM-CSF and cultured in tissue culture flasks at 37 ºC and 5% CO2 for 5 days, to allow differentiation into dendritic cells. On day 5, the cells were seeded in the same medium at 20,000 cells/well in 96-well U bottom plates, followed by addition of the hydrolysate samples including extensively hydrolyzed casein and a greater than 500 Da fraction thereof at a final concentration of 0.007, 0.02, 0.08 and 0.2 % m/v. Dexamethasone ("Dex") was used at a final concentration of 1 µg/ml as a positive control for inhibition of cytokine production. All samples were tested in biological duplicate.

On day 6, a refreshment of the cells was performed with hydrolysate samples in medium (RPMI + 10% FBS + pen/strep) without GM-CSF and IL-4. One hour after hydrolysate addition, CD40L + enhancer (Alexis) was added to a final concentration of 0.5 µg/ml. The cells were then incubated at 37 ºC and 5% CO2 for 24 hours and subsequently supernatants were collected and stored at -20°C until quantification of cytokines using the Meso Scale Discovery pro-inflammatory cytokine multiplex assay according to the manufactures instructions.

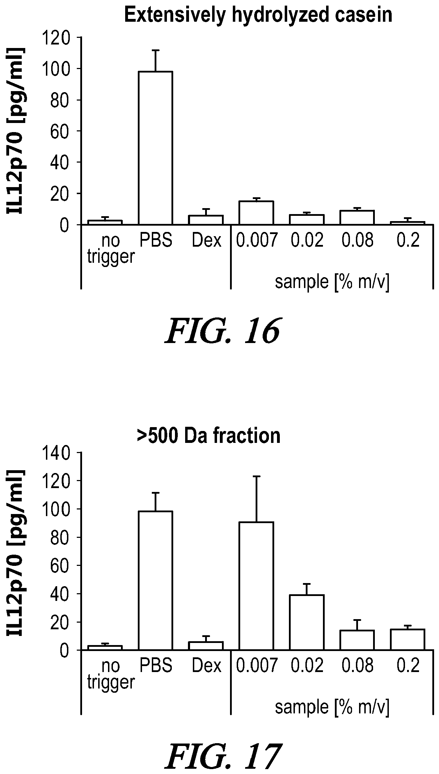

As shown in Figs. 16-17, pro-inflammatory cytokine secretion of Interleukin-12p70 (IL12p70) by activated primary human dendritic cells is inhibited by extensively hydrolyzed casein and a greater than 500Da fraction of the extensive casein hydrolysate.

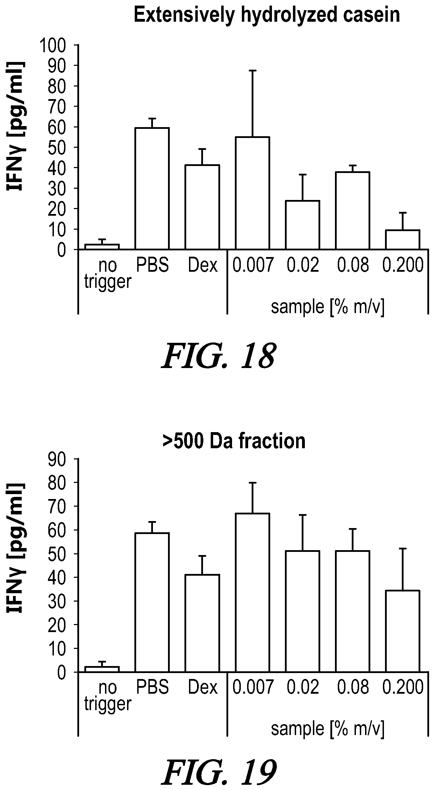

As shown in Figs. 18-19, pro-inflammatory cytokine secretion of Interferon-gamma (IFNý) by activated primary human dendritic cells is inhibited by extensively hydrolyzed casein and a greater than 500 Da fraction of the extensive casein hydrolysate.

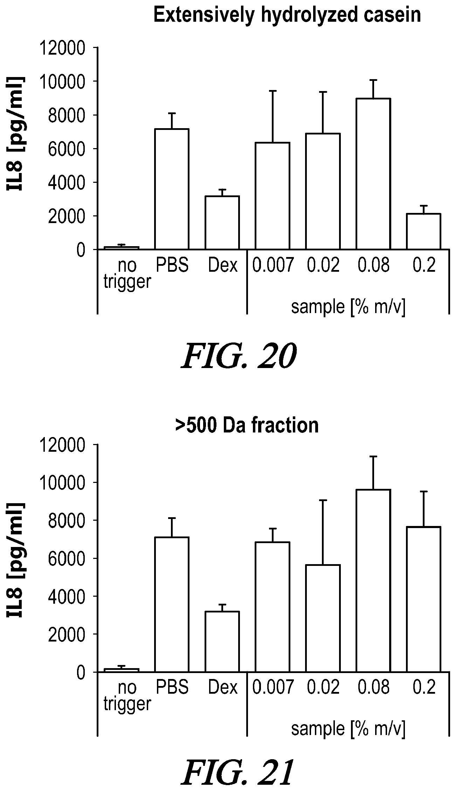

As shown in Figs. 20-21, pro-inflammatory cytokine secretion of Interleukin-8 (IL8) by activated primary human dendritic cells is inhibited by extensively hydrolyzed casein and a greater than 500 Da fraction of the extensive casein hydrolysate.

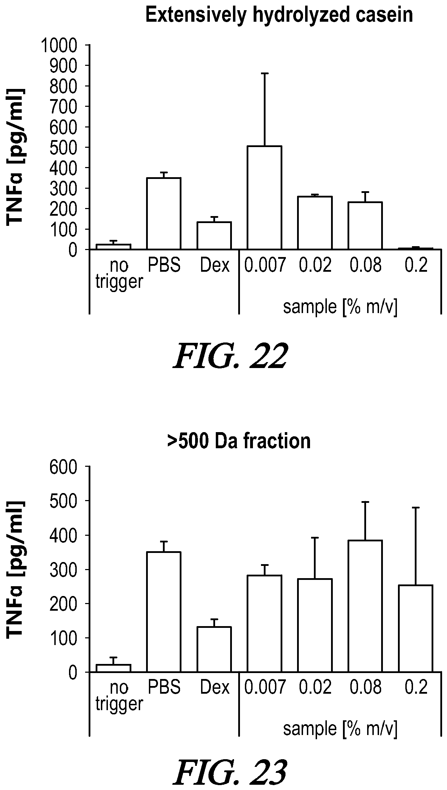

As shown in Figs. 22-23, pro-inflammatory cytokine secretion of Tumor Necrosis Factor-alpha (TNFα) by activated primary human dendritic cells is inhibited by extensively hydrolyzed casein and a greater than 500 Da fraction of the extensive casein hydrolysate.

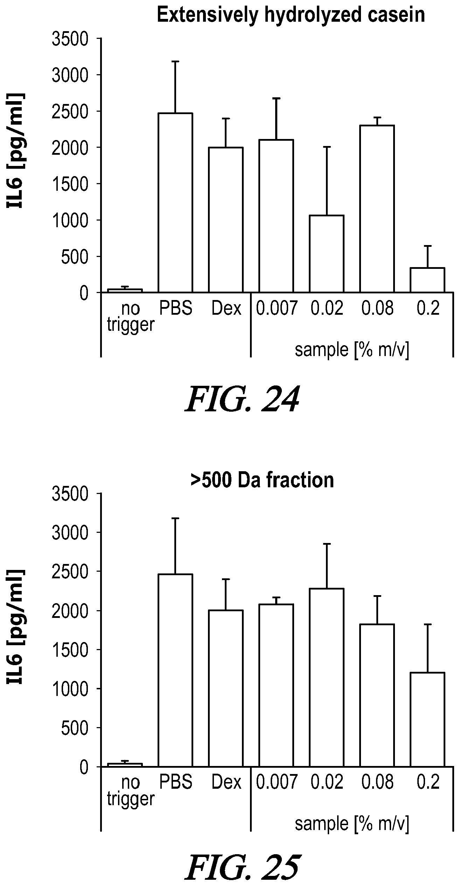

As shown in Figs. 24-25, pro-inflammatory cytokine secretion of Interleukin-6 (IL6) by activated primary human dendritic cells is inhibited by extensively hydrolyzed casein and a greater than 500 Da fraction of the extensive casein hydrolysate.

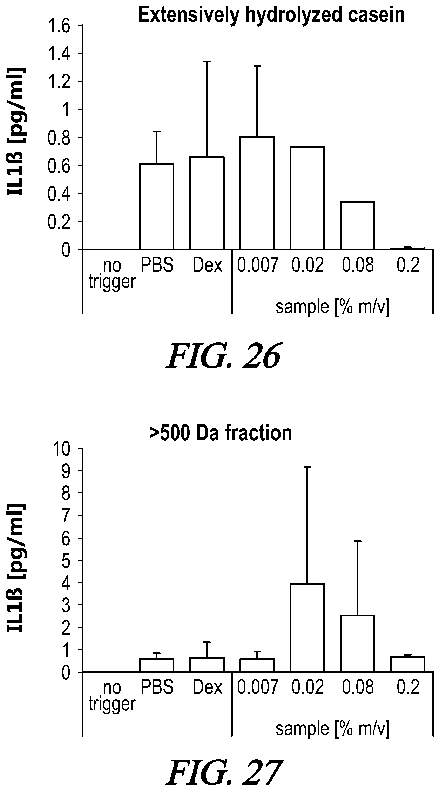

As shown in Figs. 26-27, pro-inflammatory cytokine secretion of Interleukin-lbeta (IL1β) by activated primary human dendritic cells is inhibited by extensively hydrolyzed casein and a greater than 500 Da fraction of the extensive casein hydrolysate.

### Example 3

Example 3 is directed to the inhibition of pro-inflammatory cytokine Tumor Necrosis Factor-alpha ("TNFa") in a primary human macrophage assay by extensively hydrolyzed casein hydrolysate and a greater than 500 Da fraction thereof. Isolated monocytes were suspended in RPMI medium supplemented with 10 % heat-inactivated fetal bovine serum (FBS) and penicillin/streptavidin. Recombinant human M-CSF was added to a final concentration of 100 ng/ml and cells were incubated at 37 ºC and 5% CO2 for 5 days.

On day 5, the hydrolysate samples including extensively hydrolyzed casein and a greater than 500 Da fraction thereof at a final concentration of 0.007, 0.02, 0.08 and 0.2 % m/v were added in RPMI medium with M-CSF. On day 6, the medium including the hydrolysate samples was removed and cells were refreshed with hydrolysate samples in medium without M-CSF. One hour after hydrolysate addition, LPS (E. Coli (0111:84) was added to a final concentration of 10 ng/ml. The cells were then incubated at 37 ºC and 5% CO2 for 24 hours and subsequently supernatants were collected and stored at -20 °C until quantification of TNFa using the Meso Scale Discovery TNFa assay according to the manufactures instructions

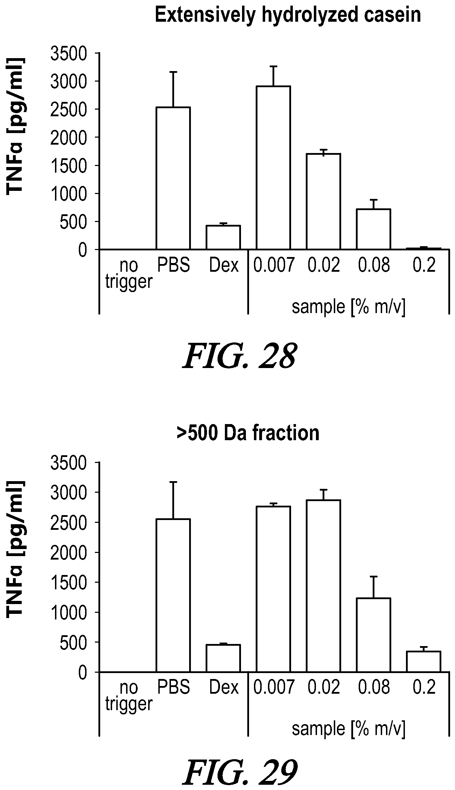

As shown in Figs. 28-29, pro-inflammatory cytokine secretion of Tumor Necrosis Factor-alpha (TNFα) of activated primary human macrophages is inhibited by extensively hydrolyzed casein and a greater than 500 Da fraction of the extensive casein hydrolysate.

### Example 4

Example 4 is directed to the evaluation of pro-inflammatory cytokine messenger RNA expression in the ileum of Non-Obese Diabetic (NOD) mice. Briefly, age-matched female BALB/c mice were used as the healthy control mice. BALB/c mice were kept on regular chow if not otherwise stated, whereas NOD mice were kept either on regular chow or extensively hydrolyzed casein (CH)-containing dietary formulation starting at the time of weaning. RNA isolation from ileum samples was performed by an RNA isolation kit. cDNA was synthesized and cytokine expression was assessed by quantitative real-time PCR. Absolute copy numbers of RNA (molecules/µg) were calculated by running standards and glyceraldehyde-3-phosphate dehydrogenase (GAPDH) as loading control.

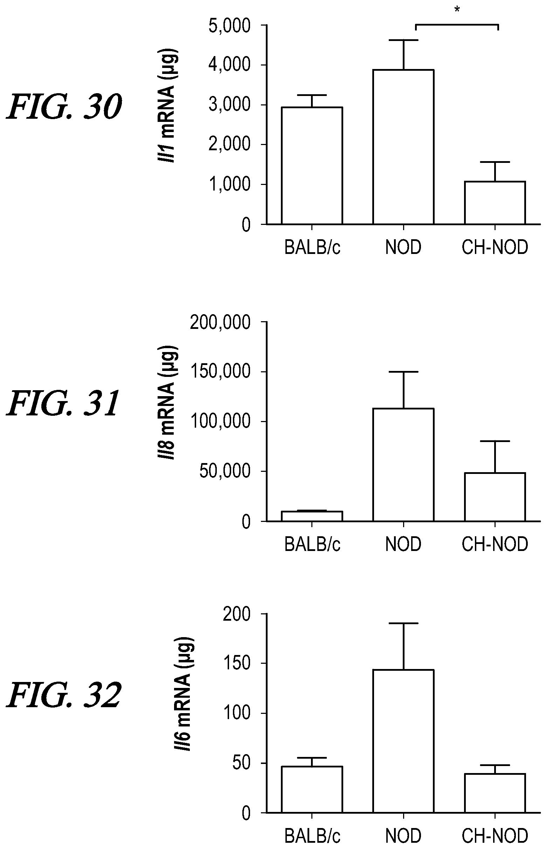

As can be seen in Figs. 30-33, pro-inflammatory cytokine secretion of IL1, IL6, IL18 and IL17 in the ileum of NOD mice is elevated as compared to age-matched healthy (BALB/C) control mice. These elevated levels are decreased in NOD mice fed an extensively hydrolyzed casein diet.

### Example 5

Example 5 is directed to the evaluation of pro-inflammatory cytokine profiles from T cell isolated from gut-draining lymph nodes of Non-Obese Diabetic (NOD) mice. Briefly, age-matched female BALB/c mice were used as the healthy control mice. BALB/c mice were kept on regular chow if not otherwise stated, whereas NOD mice were kept either on regular chow or an extensively hydrolyzed casein (CH)-containing dietary formulation starting at the time of weaning.

Isolated mesenteric lymph node cells from each group of mice, were stimulated with platebound anti-CD3 in complete medium (DMEM with 10% (vol./vol.) FBS, L-glutamine 2 mmol/l,100 U/ml penicillin plus streptomycin). Supernatant fractions were collected 48 h later for cytokine analysis with multiplex cytokine bead arrays according to the manufactures instructions.

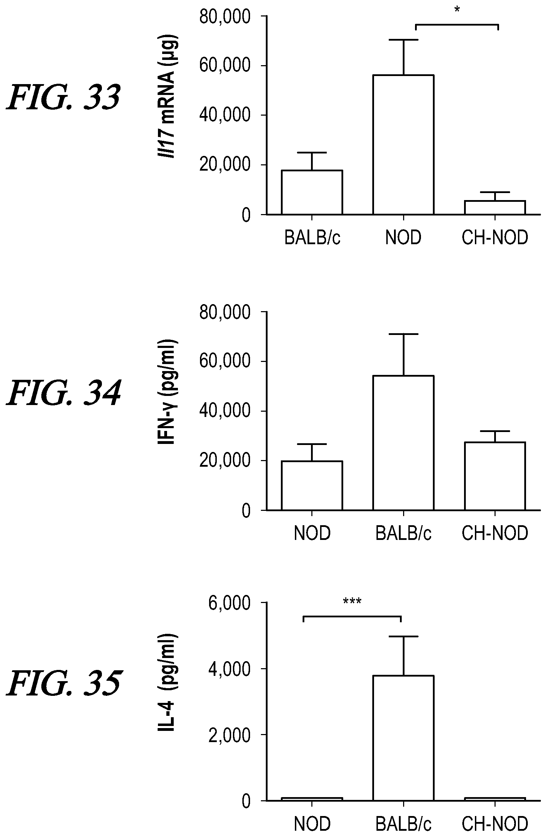

As shown in Figs. 34-35, secretion of pro-inflammatory cytokines IFNy, IL4, and IL17 of gut-draining lymph node T cells from NOD mice is elevated as compared to age-matched healthy (BALB/C) control mice. These elevated levels were decreased in NOD mice by an extensively hydrolyzed casein diet.

### Example 6

Example 6 is directed to cytokine secretion analysis in a murine macrophage cell line. The J774A.1 murine macrophage cell line was maintained in RPMI-1640 supplemented with 10% (v/v) fetal calf serum, 50 IU/ml penicillin and 0.5mg/ml streptomycin. Cells were incubated at 370C with 5% CO2. Casein hydrolysate samples including extensively hydrolysed casein and a greater than 500Da fraction thereof were added to sterile water at a concentration of 100mg/ml and filter sterilised through 0.2µm nylon filters. As an additional control also non-filtered sterilized extensively hydrolysed casein was tested. Cells were plated at 1x106 cells/ml and incubated in triplicate with a final concentration of 1 mg/ml hydrolysate for 1 hour. Cells were then either treated with 100ng/ml LPS or left untreated for 24 hours before collecting supernatants by centrifugation and storing at -20°C. Supernatants were assessed for each cytokine concentration using individual ELISA assays for IL-1β, IL-6, IL12p40, and TNF-α according to the manufactures instructions. Samples were tested in triplicate and significant differences were calculated using the ordinary one-way ANOVA analysis on graphpad prism 6 software. p<0.05 was considered significant.

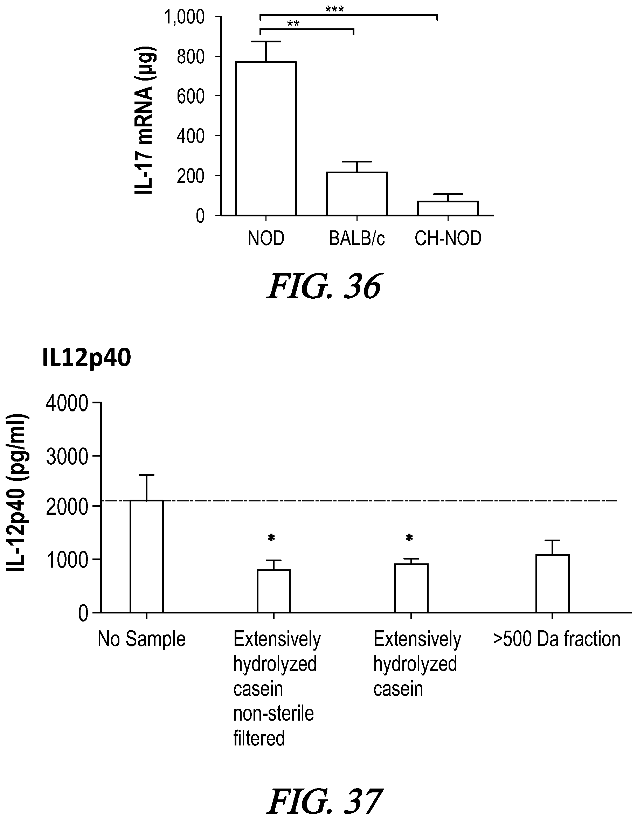

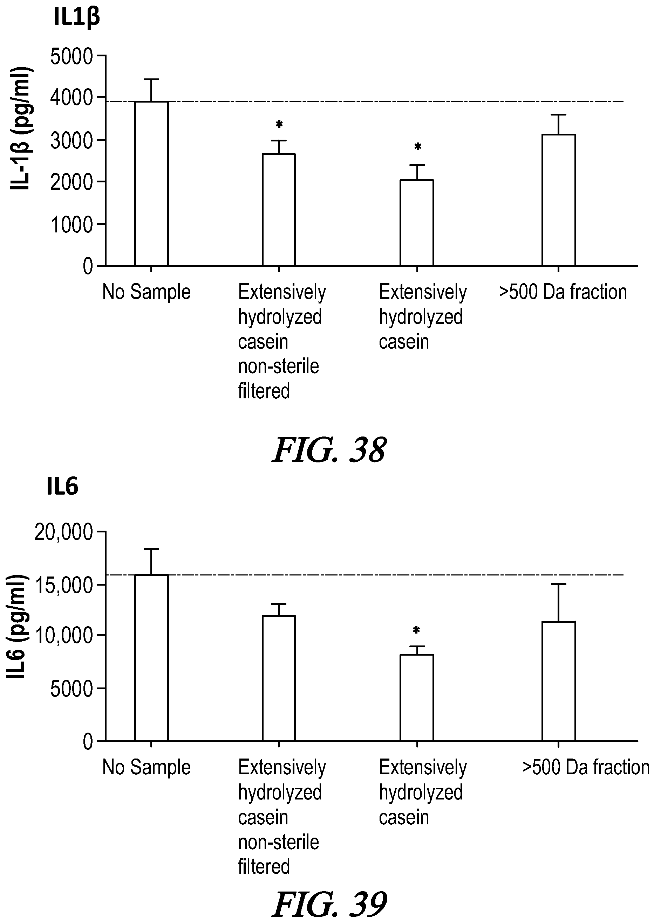

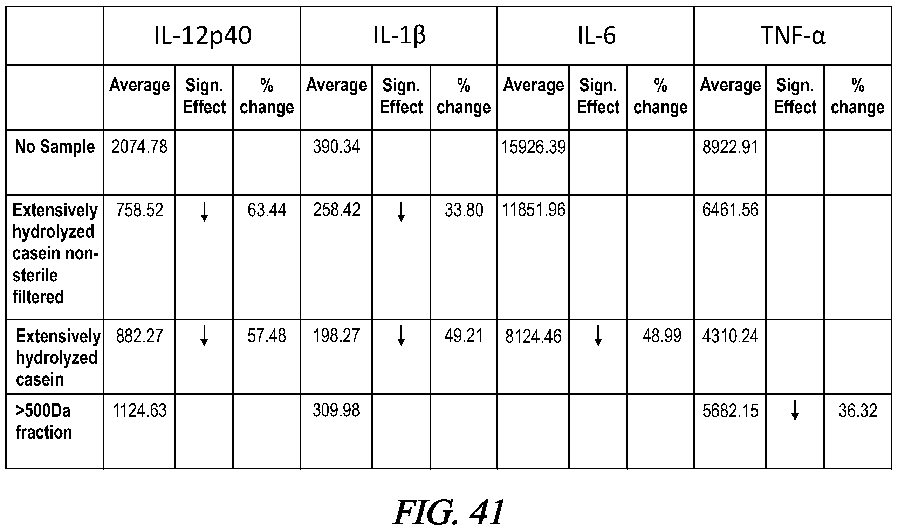

As can be seen from Figs. 37-38 pro-inflammatory cytokine secretion IL-12p40 and IL-1β from mouse macrophages stimulated with LPS was decreased by extensively hydrolyzed casein, both with or without sterile filtration prior to incubation of the cells (* p<0.05). Secretion of the pro-inflammatory cytokine IL6 was significantly decreased by extensively hydrolyzed casein (* p<0.05). Secretion of the pro-inflammatory cytokine TNFa was significantly decreased by the greater than 500 Da fraction of the extensive casein hydrolysate (* p<0.05). The results are also shown in Table format in Fig. 41.

## Claims

1. A nutritional composition, comprising a lipid or a fat phase, protein source and a casein hydrolysate for use in the treatment of an inflammatory disease, wherein the inflammatory
disease is selected from the group consisting of type 1 diabetes, Crohn's disease, Ulcerative colitis, Metabolic syndrome, and Coeliac disease; in a subject, and
wherein the nutritional composition comprises about 0.1 to about 1 g/100 kcal of a prebiotic, and wherein the prebiotic composition comprises at least 20% of an oligosaccharide and wherein the hydrolysate consists of peptides with a molecular weight of more than 500 Da;
and wherein the hydrolysate comprises a peptide with SEQ ID NO: 1 and at least two peptides selected from the group consisting of SEQ ID NO: 2-68.

2. The composition for use according to claim 1, wherein the hydrolysate comprises at least three peptides, preferably at least four peptides and most preferably at least five peptides selected from the group consisting of SEQ ID NO: 2-68.

3. The composition for use according to claim 1, wherein the nutritional composition further comprises about 5 to about 100 mg/100 kcal of a source of long chain polyunsaturated fatty acids which comprises docosahexaenoic acid.

4. The composition for use according to claim 3, wherein the nutritional composition further comprises arachidonic acid.

5. The composition for use according to claim 1, wherein the subject is a human child or juvenile.

6. The composition for use according to claim 1, wherein the composition additionally comprises one or more of carbohydrates, nucleic acids, lipids, minerals, anabolic nutrients, vitamins, antioxidants, probiotic bacterial strains and lipotropic agents.

## Patentansprüche

1. Nährstoffzusammensetzung, umfassend eine Lipid- oder Fettphase, eine Proteinquelle und ein Kaseinhydrolysat zur Verwendung bei der Behandlung einer Entzündungskrankheit, wobei die Entzündungskrankheit ausgewählt ist aus der Gruppe, bestehend aus Diabetes Typ 1, Morbus Crohn, Colitis ulcerosa, metabolischem Syndrom und Zöliakie in einem Probanden, und
wobei die Nährstoffzusammensetzung etwa 0,1 bis etwa 1 g/100 kcal eines Präbiotikums umfasst und wobei die präbiotische Zusammensetzung mindestens 20 % eines Oligosaccharids umfasst, und wobei das Hydrolysat aus Peptiden mit einem Molekulargewicht von mehr als 500 Da besteht;
und wobei das Hydrolysat ein Peptid mit SEQ ID Nr.: 1 und mindestens zwei Peptide, ausgewählt aus der Gruppe bestehend aus SEQ ID Nr.: 2-68, umfasst.

2. Zusammensetzung zur Verwendung nach Anspruch 1, wobei das Hydrolysat mindestens drei Peptide, vorzugsweise mindestens vier Peptide und am meisten bevorzugt mindestens fünf Peptide umfasst, ausgewählt aus der Gruppe bestehend aus SEQ ID Nr.: 2-68.

3. Zusammensetzung zur Verwendung nach Anspruch 1, wobei die Nährstoffzusammensetzung ferner etwa 5 bis etwa 100 mg/100 kcal einer Quelle langkettiger, mehrfach ungesättigter Fettsäuren umfasst, die Docosahexaensäure umfasst.

4. Zusammensetzung zur Verwendung nach Anspruch 3, wobei die Nährstoffzusammensetzung ferner Arachidonsäure umfasst.

5. Zusammensetzung zur Verwendung nach Anspruch 1, wobei der Proband ein menschliches Kind oder Jugendlicher ist.

6. Zusammensetzung zur Verwendung nach Anspruch 1, wobei die Zusammensetzung zusätzlich eines oder mehrere von Kohlenhydraten, Nukleinsäuren, Lipiden, Mineralien, anabolen Nährstoffen, Vitaminen, Antioxidationsmitteln, probiotischen Bakterienstämmen und lipotropen Mitteln umfasst.

## Revendications

1. Composition nutritionnelle, comprenant un lipide ou une phase grasse, une source de protéines et un hydrolysat de caséine pour l'utilisation dans le traitement d'une maladie inflammatoire, la maladie inflammatoire étant choisie dans le groupe comprenant le diabète de type 1, la maladie de Crohn, la colite ulcéreuse, le syndrome métabolique et la maladie cœliaque ; chez un sujet, et
la composition nutritionnelle comprenant environ 0,1 à environ 1 g/100 kcal d'un prébiotique, et la composition prébiotique comprenant au moins 20 % d'un oligosaccharide et l'hydrolysat étant constitué de peptides ayant un poids moléculaire supérieur à 500 Da ;
et l'hydrolysat comprenant un peptide avec SEQ ID NO: 1 et au moins deux peptides choisis dans le groupe comprenant SEQ ID NO: 2 à 68.

2. Composition à utiliser selon la revendication 1, l'hydrolysat comprenant au moins trois peptides, de préférence au moins quatre peptides et idéalement au moins cinq peptides choisis dans le groupe comprenant SEQ ID NO: 2 à 68.

3. Composition à utiliser selon la revendication 1, la composition nutritionnelle comprenant en outre environ 5 à environ 100 mg/100 kcal d'une source d'acides gras polyinsaturés à longue chaîne qui comprend de l'acide docosahexaénoïque.

4. Composition à utiliser selon la revendication 3, la composition nutritionnelle comprenant en outre de l'acide arachidonique.

5. Composition à utiliser selon la revendication 1, le sujet étant un enfant ou une jeune personne.

6. Composition à utiliser selon la revendication 1, la composition comprenant en outre un ou plusieurs glucides, acides nucléiques, lipides, minéraux, nutriments anabolisants, vitamines, antioxydants, souches bactériennes probiotiques et agents lipotropes.


### SEQUENCE LISTING

<110> Mead Johnson Nutritional Company Ltd. (Asai Pacific)
<120> Reducing Proinflammatory Response
<130> MJE00378NP
<160> 68
<170> PatentIn version 3.5
<210> 1
   <211> 6
   <212> PRT
   <213> BOVINE
<400> 1
   Ile Pro Asn Pro Ile Gly
   1               5
<210> 2
   <211> 9
   <212> PRT
   <213> BOVINE
<400> 2
   Ile Gly Ser Glu Ser Thr Glu Asp Gln
   1               5
<210> 3
   <211> 7
   <212> PRT
   <213> BOVINE
<400> 3
   Asp Lys Thr Glu Ile Pro Thr
   1               5
<210> 4
   <211> 4
   <212> PRT
   <213> BOVINE
<400> 4
   Ile Val Pro Asn
   1
<210> 5
   <211> 6
   <212> PRT
   <213> BOVINE
<400> 5
   Leu Glu Asp Ser Pro Glu
   1               5
<210> 6
   <211> 6
   <212> PRT
   <213> BOVINE
<400> 6
   Asn Gln Glu Gln Pro Ile
   1 5
<210> 7
   <211> 5
   <212> PRT
   <213> BOVINE
<400> 7
   Asn Val Pro Gly Glu
   1 5
<210> 8
   <211> 6
   <212> PRT
   <213> BOVINE
<400> 8
   Pro Phe Pro Gly Pro Ile
   1 5
<210> 9
   <211> 5
   <212> PRT
   <213> BOVINE
<400> 9
   Thr Glu Asp Glu Leu
   1 5
<210> 10
   <211> 4
   <212> PRT
   <213> BOVINE
<400> 10
   Val Pro Ser Glu
   1
<210> 11
   <211> 6
   <212> PRT
   <213> BOVINE
<400> 11
   Tyr Pro Phe Pro Gly Pro
   1 5
<210> 12
   <211> 5
   <212> PRT
   <213> BOVINE
<400> 12
   Tyr Pro Ser Gly Ala
   1               5
<210> 13
   <211> 6
   <212> PRT
   <213> BOVINE
<400> 13
   Phe Pro Gly Pro Ile Pro
   1               5
<210> 14
   <211> 11
   <212> PRT
   <213> BOVINE
<400> 14
   Met His Gln Pro His Gln Pro Leu Pro Pro Thr
   1               5                   10
<210> 15
   <211> 9
   <212> PRT
   <213> BOVINE
<400> 15
   Tyr Pro Phe Pro Gly Pro Ile Pro Asn
   1               5
<210> 16
   <211> 5
   <212> PRT
   <213> BOVINE
<400> 16
   Asp Met Glu Ser Thr
   1               5
<210> 17
   <211> 7
   <212> PRT
   <213> BOVINE
<400> 17
   Phe Pro Gly Pro Ile Pro Asn
   1 5
<210> 18
   <211> 5
   <212> PRT
   <213> BOVINE
<400> 18
   Ile Pro Asn Pro Ile
   1 5
<210> 19
   <211> 6
   <212> PRT
   <213> BOVINE
<400> 19
   Met Glu Ser Thr Glu Val
   1 5
<210> 20
   <211> 6
   <212> PRT
   <213> BOVINE
<400> 20
   Pro Gly Pro Ile Pro Asn
   1 5
<210> 21
   <211> 8
   <212> PRT
   <213> BOVINE
<400> 21
   Pro His Gln Pro Leu Pro Pro Thr
   1 5
<210> 22
   <211> 4
   <212> PRT
   <213> BOVINE
<400> 22
   Pro Asn Pro Ile
   1
<210> 23
   <211> 7
   <212> PRT
   <213> BOVINE
<400> 23
   Ser Lys Asp Ile Gly Ser Glu
   1 5
<210> 24
   <211> 8
   <212> PRT
   <213> BOVINE
<400> 24
   Tyr Pro Phe Pro Gly Pro Ile Pro
   1 5
<210> 25
   <211> 8
   <212> PRT
   <213> BOVINE
<400> 25
   Ala Ile Asn Pro Ser Lys Glu Asn
   1 5
<210> 26
   <211> 5
   <212> PRT
   <213> BOVINE
<400> 26
   Ala Pro Phe Pro Glu
   1 5
<210> 27
   <211> 6
   <212> PRT
   <213> BOVINE
<400> 27
   Asp Ile Gly Ser Glu Ser
   1 5
<210> 28
   <211> 4
   <212> PRT
   <213> BOVINE
<400> 28
   Asp Met Pro Ile
   1
<210> 29
   <211> 4
   <212> PRT
   <213> BOVINE
<400> 29
   Asp Val Pro Ser
   1
<210> 30
   <211> 3
   <212> PRT
   <213> BOVINE
<400> 30
   Glu Asp Ile
   1
<210> 31
   <211> 3
   <212> PRT
   <213> BOVINE
<400> 31
   Glu Leu Phe
   1
<210> 32
   <211> 3
   <212> PRT
   <213> BOVINE
<400> 32
   Glu Met Pro
   1
<210> 33
   <211> 7
   <212> PRT
   <213> BOVINE
<400> 33
   Glu Thr Ala Pro Val Pro Leu
   1 5
<210> 34
   <211> 4
   <212> PRT
   <213> BOVINE
<400> 34
   Gly Pro Phe Pro
   1
<210> 35
   <211> 4
   <212> PRT
   <213> BOVINE
<400> 35
   Gly Pro Ile Val
   1
<210> 36
   <211> 8
   <212> PRT
   <213> BOVINE
<400> 36
   Ile Gly Ser Ser Ser Glu Glu Ser
   1               5
<210> 37
   <211> 9
   <212> PRT
   <213> BOVINE
<400> 37
   Ile Gly Ser Ser Ser Glu Glu Ser Ala
   1               5
<210> 38
   <211> 6
   <212> PRT
   <213> BOVINE
<400> 38
   Ile Asn Pro Ser Lys Glu
   1               5
<210> 39
   <211> 9
   <212> PRT
   <213> BOVINE
<400> 39
   Ile Pro Pro Leu Thr Gln Thr Pro Val
   1               5
<210> 40
   <211> 4
   <212> PRT
   <213> BOVINE
<400> 40
   Ile Thr Ala Pro
   1
<210> 41
   <211> 7
   <212> PRT
   <213> BOVINE
<400> 41
   Lys His Gln Gly Leu Pro Gln
   1               5
<210> 42
   <211> 5
   <212> PRT
   <213> BOVINE
<400> 42
   Leu Asp Val Thr Pro
   1               5
<210> 43
   <211> 5
   <212> PRT
   <213> BOVINE
<400> 43
   Leu Pro Leu Pro Leu
   1               5
<210> 44
   <211> 5
   <212> PRT
   <213> BOVINE
<400> 44
   Asn Ala Val Pro Ile
   1               5
<210> 45
   <211> 5
   <212> PRT
   <213> BOVINE
<400> 45
   Asn Glu Val Glu Ala
   1               5
<210> 46
   <211> 3
   <212> PRT
   <213> BOVINE
<400> 46
   Asn Leu Leu
   1
<210> 47
   <211> 5
   <212> PRT
   <213> BOVINE
<400> 47
   Pro Ile Thr Pro Thr
   1 5
<210> 48
   <211> 6
   <212> PRT
   <213> BOVINE
<400> 48
   Pro Asn Ser Leu Pro Gln
   1 5
<210> 49
   <211> 6
   <212> PRT
   <213> BOVINE
<400> 49
   Pro Asn Ser Leu Pro Gln
   1 5
<210> 50
   <211> 7
   <212> PRT
   <213> BOVINE
<400> 50
   Pro Gln Asn Ile Pro Pro Leu
   1 5
<210> 51
   <211> 6
   <212> PRT
   <213> BOVINE
<400> 51
   Pro Val Leu Gly Pro Val
   1 5
<210> 52
   <211> 4
   <212> PRT
   <213> BOVINE
<400> 52
   Pro Val Pro Gln
   1
<210> 53
   <211> 5
   <212> PRT
   <213> BOVINE
<400> 53
   Pro Val Val Val Pro
   1 5
<210> 54
   <211> 6
   <212> PRT
   <213> BOVINE
<400> 54
   Pro Val Val Val Pro Pro
   1 5
<210> 55
   <211> 11
   <212> PRT
   <213> BOVINE
<400> 55
   Ser Ile Gly Ser Ser Ser Glu Glu Ser Ala Glu
   1 5 10
<210> 56
   <211> 7
   <212> PRT
   <213> BOVINE
<400> 56
   Ser Ile Ser Ser Ser Glu Glu
   1 5
<210> 57
   <211> 11
   <212> PRT
   <213> BOVINE
<400> 57
   Ser Ile Ser Ser Ser Glu Glu Ile Val Pro Asn
   1 5 10
<210> 58
   <211> 6
   <212> PRT
   <213> BOVINE
<400> 58
   Ser Pro Pro Glu Ile Asn
   1 5
<210> 59
   <211> 7
   <212> PRT
   <213> BOVINE
<400> 59
   Ser Pro Pro Glu Ile Asn Thr
   1 5
<210> 60
   <211> 7
   <212> PRT
   <213> BOVINE
<400> 60
   Thr Asp Ala Pro Ser Phe Ser
   1 5
<210> 61
   <211> 6
   <212> PRT
   <213> BOVINE
<400> 61
   Val Ala Thr Glu Glu Val
   1 5
<210> 62
   <211> 5
   <212> PRT
   <213> BOVINE
<400> 62
   Val Leu Pro Val Pro
   1 5
<210> 63
   <211> 4
   <212> PRT
   <213> BOVINE
<400> 63
   Val Pro Gly Glu
   1
<210> 64
   <211> 6
   <212> PRT
   <213> BOVINE
<400> 64
   Val Pro Gly Glu Ile Val
   1               5
<210> 65
   <211> 6
   <212> PRT
   <213> BOVINE
<400> 65
   Val Pro Ile Thr Pro Thr
   1               5
<210> 66
   <211> 9
   <212> PRT
   <213> BOVINE
<400> 66
   Val Val Pro Pro Phe Leu Gln Pro Glu
   1               5
<210> 67
   <211> 5
   <212> PRT
   <213> BOVINE
<400> 67
   Val Val Val Pro Pro
   1               5
<210> 68
   <211> 5
   <212> PRT
   <213> BOVINE
<400> 68
   Tyr Pro Val Glu Pro
   1               5